# FASCICULE DE BREVET EUROPEEN

(11) **EP 1 517 916 B1**
(45) Date de publication et mention de la délivrance du brevet: **09.12.2009**
(21) Numéro de dépôt: 03761672.9
(22) Date de dépôt: 01.07.2003
(51) Int. Cl.: A61K 38/17, A23L 3/3463, A23L 3/3535

(54) **SPHÉNISCINES ET PEPTIDES ANALOGUES ANTIMICROBIENS POUR LA CONSERVATION DES ALIMENTS**
SPHENISCINS UND ANALOGE ANTIMIKROBIELLE PEPTIDE ZUR KONSERVIERUNG VON LEBENSMITTELN
SPHENISCINS AND ANALOGUE PEPTIDES HAVING ANTIMICROBIAL PROPERTIES FOR USE IN PRESERVING FOODS

(30) Priorité: 01.07.2002 FR 0208207
(43) Date de publication de la demande: 30.03.2005
(73) Titulaire: Centre National de La Recherche Scientifique-CNRS, 75794 Paris (FR)
(72) Inventeur: BULET, Philippe, 74100 Anemasse (FR); THOUZEAU, Cécile, 67300 Schiltigheim (FR); LE MAHO, Yvon, F-67800 Hoenheim (FR)
(74) Mandataire: Breese, Pierre
(86) Numéro de dépôt international: PCT/FR2003/002041
(87) Numéro de publication internationale: WO 2004/003006

(56) Documents cités:
- WO-A-02/09738
- US-A- 5 635 594
- US-B1- 6 399 370
- SCHUTTE: "Discovery of five conserved beta-defensin gene clusters using a computational search strategy" PROCEEDINGS OF THE NATIONAL ACADEMY OF SCIENCES, U.S.A , vol. 99, 19 février 2002 (2002-02-19), pages 2129-2133, XP002250229
- BACHÈRE ET AL: "Penaeidins, antimicrobial peptides of shrimp: a comparison with other effectors of innate immunity" AQUACULTURE, vol. 191, 2000, pages 71-88, XP002250230
- DIMARCQ ET AL: "Cysteine-rich antimicrobial peptides in invertebrates" BIOPOLYMERS, vol. 47, 1998, pages 465-477, XP002250231
- ZHAO C. ET AL: 'Gallinacin-3, an inducible epithelial beta-defensin in the chicken' INFECTION AND IMMUNITY vol. 69, no. 4, Avril 2001, pages 2684 - 2691, XP001167116
- BROCKUS C.W. ET AL: 'Characterization of beta-defensin prepropeptide mRNA from chicken and turkey bone marrow' ANIMAL GENETICS vol. 29, 1998, pages 283 - 289, XP001167115
- BAUER F. ET AL: 'Structure determination of human and murine beta-defensins reval structural conservation in the absence of significant sequence similarity' PROTEIN SCIENCE vol. 10, 2001, pages 2470 - 2479, XP002379594
- SELSTED M.E. ET AL: 'Purification, primary structures, and antibacterial activities of beta-defensins, a new family of antimicrobial peptides form bovine neutrophils' THE JOURNAL OF BIOLOGICAL CHEMISTRY vol. 268, no. 9, Mars 1998, pages 6641 - 6648, XP002967191

## Description

La présente invention concerne le domaine de la conservation des aliments et de la lutte contre des infections microbiennes, bactériennes et/ou fongiques chez 1 l'homme, l'animal ou les plantes. Elle vise aux peptides antimicrobiens possédant une structure de bêta-defensin, en particulier aux sphéniscines et leurs analogues, ainsi que les compositions les contenant pour prévenir et/ou traiter des infections par des agents pathogènes comme les microbes, les bactéries et champignons ainsi que pour la conservation des aliments.

Il a été rapporté dans l'art antérieur que le mâle de Manchot Royal est capable, lors de son séjour à terre pour assurer la dernière partie de l'incubation, de garder de la nourriture dans son estomac pendant plus de deux semaines (Gauthier-Clerc *et al.,* 2000). Lui-même jeûne et vit sur ses réserves corporelles.

Il n'existe pas à ce jour de situation similaire connue de stockage de nourriture pendant plusieurs semaines chez un vertébré supérieur. L'état de conservation de cette nourriture est remarquable, sa masse et sa valeur énergétique n'étant pas modifiées (Gauthier-Clerc *et al.,* 2002). Ces observations sur l'état de conservation de la nourriture suggèrent fortement un contrôle de la flore bactérienne présente dans le contenu stomacal. Ce contrôle de la flore bactérienne permettrait de réduire les dégradations de l'alimentation stockée. Ce contrôle pourrait se faire via la production de substances à activité antimicrobienne,

En effet, la littérature relate l'existence de substances à activité antimicrobienne dans le tractus gastro-intestinal des Vertébrés. Une grande partie de ces substances est de nature peptidique, notamment des magainines, brévinines et buforines chez les amphibiens (Moore *et al*., 1991 ; Minn *et al*., 1998 ; Wang *et al*., 1998), des lactoferricines et des défensines (α, β) chez les mammifères, dont l'homme (Jones & Bevins, 1992; Zhao *et al*., 1999; O'Neil *et al*., 2000). Chez les oiseaux, des défensines ont été trouvées dans d'autres parties du tractus digestif, notamment la langue, l'oesophage et l'intestin (Zhao *et al*. 2001). De plus de nombreux peptides antimicrobiens sont également présents dans les épithélia de surface (pour revue voir Schröder 1999).

Les inventeurs ont maintenant mis en évidence la présence de peptides antimicrobiens dans le contenu stomacal des Manchots Royaux et ont montré l'implication de ces peptides à activité antimicrobiennes dans le phénomène de conservation du bol alimentaire lors du jeûne d'incubation chez le mâle de Manchot Royal. Ces travaux de recherche ont permis de définir de nouveaux peptides qui seront désignés ci-après « sphéniscines » d'après la famille des Sphéniscidés à laquelle appartient le Manchot Royal.

L'invention a donc pour objet un peptide ou un analogue de sphéniscine qui présente une activité anti-bactérienne et comprend trois liaisons intra-moléculaires, et répond à la formule (II) suivante :

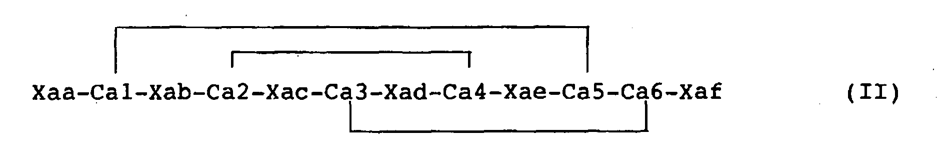

Dans laquelle :
- Xaa répond à la formule suivante : -Xaa1-Xaa2-(SEQ ID NO.2), où Xaal représente un groupe -NH₂ ou un reste peptidique de 1 à 13 acides aminés, et Xaa2 est Ser-Phe-Gly-Leu;
- Xab répond à la formule suivante : -Xab1-Xab2-Xab3-Xab4-Xab5- (SEQ ID NO.3), et Xab3 représente un reste peptidique répondant à la formule suivante : -Xab3.1-Xab3.2-(SEQ ID NO.4),
- Xac répond à la formule suivante : -Xac1-Xac2-Xac3-Xac4- (SEQ ID NO.5), dans laquelle Xac2 est His ou Arg
- Xad à la séquence suivante : -Xad1-Xad2-Xad3-xad4-xad5-xad6-Xad7-Xad8-Xad9- (SEQ ID NO.6), laquelle Xad2 est Pro ou Phe et Xad3 est Pro ;
- Xae répond à la séquence suivante : -Xae1-Xae2-Xae3-Xae4-Xae5- (SEQ ID NO.7), dans laquelle Xae2 est Gln ou Arg et Xae5 est Gln ;
- Xaf répond à la séquence suivante : -Xaf1-Xaf2-Xaf3-Xaf4- (SEQ ID NO.8) et Xaf2 et/ou Xaf3 est Val; et - Cal, Ca2, Ca3, Ca4, Ca5 et Ca6, sont des acides aminés soufrés.
- Xab représente un reste peptidique de 6 acides aminés Xab répond à la formule suivante : -Xab1-Xab2-Xab3-Xab4-Xab5- (SEQ ID NO.3), où ceux des Xab1, Xab2, Xab4 et Xab5 présents, identiques ou différents, sont choisis dans le groupe comprenant les acides aminés basiques, les acides aminés polaires chargés négativement, les acides aminés polaires petits non chargés, les acides aminés polaires larges non chargés et les acides aminés hydrophobes ou apolaires, et Xab3 représente un reste peptidique répondant à la formule suivante : -Xab3.1-Xab3.2-(SEQ ID NO.4), où ceux des Xab3.1 et Xab3.2 présents, identiques ou différents, sont choisis dans le groupe comprenant les acides aminés basiques, les acides aminés polaires chargés négativement, les acides aminés polaires petits non chargés, les acides aminés polaires larges non chargés et les acides aminés hydrophobes ou apolaires ;
- Xac représente un reste peptidique comprenant 4 acides aminés Xac répond à la formule suivante : -Xacl-Xac2-Xac3-Xac4- (SEQ ID NO.5), où ceux des Xac1, Xac3 et Xac4 présents, identiques ou différents, sont choisis dans le groupe comprenant les acides aminés basiques, les acides aminés polaires petits non chargés, les acides aminés hydrophobes ou apolaires et les acides aminés polaires larges non chargés et Xac₂ est His ou Arg
- Xad représente un reste peptidique comprenant 9 acides aminés, Xad répond à la séquence suivante : -Xad1-Xad2-Xad3-Xad4-Xad5-Xad6-Xad7-Xad8-Xad9- (SEQ ID NO.6), où ceux des Xad1, Xad4, Xad5, Xad6, Xad7, Xad8 et Xad9 présents, identiques ou différents sont choisis dans le groupe comprenant les acides aminés basiques, les acides aminés polaires petits non chargés, les acides aminés hydrophobes ou apolaires, les acides aminés polaires larges non chargés et les acides aminés polaires chargés négativement ; dans laquelle Xad₂ est Pro ou Phe et Xad₃ est Pro
- Xae représente un reste peptidique comprenant 5 acides aminés, avantageusement Xae répond à la séquence suivante : -Xae1-Xae2-Xae3-Xae4-Xae5- (SEQ ID NO.7), où ceux des Xae1, Xae3, Xae4 et présents, identiques ou différents, sont choisis dans le groupe comprenant les acides aminés basiques, les acides aminés polaires petits non chargés, les acides aminés hydrophobes ou apolaires, les acides aminés polaires larges non chargés et les acides aminés polaires chargés négativement ; dans laquelle Xac2 est Gln ou Arq et Xaes est Gln,
- Xaf représente un reste peptidique de 4 acides aminés, avantageusement Xaf répond à la séquence suivante : -Xaf1-Xaf2-Xaf3-Xaf4- (SEQ ID NO.8), où ceux des Xaf1, et Xaf4 présents, identiques ou différents, sont choisis dans le groupe comprenant les acides aminés basiques, les acides aminés polaires petits non chargés, les acides aminés hydrophobes ou apolaires, les acides aminés polaires larges non chargés et les acides aminés polaire chargé négativement ; et Xaf2 et/ou Xaf3 est Val
- Ca1, Ca2, Ca3, Ca4, Ca5 et Ca6, identiques ou différents, Ca1, Ca2, Ca3, Ca4, Ca5 et Ca6 sont des acides aminés soufrés et préférentiellement des cystéines.
- Ca1 et -Ca5 sont liés par covalence. Préférentiellement:- Xab1 est choisi dans le groupe comprenant les acides aminés basiques, les acides aminés hydrophobes ou apolaires, les acides aminés polaires larges non chargés, de préférence Xab1 est un acide aminé basique et tout préférentiellement l'arginine (Arg).
- Xab2 est choisi dans le groupe comprenant les acides aminés hydrophobes ou apolaires, les acides aminés polaires petits non chargés, les acides aminés basiques, les acides aminés polaires larges non chargés, de préférence Xab2 est un acide aminé hydrophobe ou apolaire et tout préférentiellement la leucine (Leu).
- Xab3.2 est choisi dans le groupe comprenant les acides aminés basiques, les acides aminés polaires petits non chargés et les acides aminés hydrophobes ou apolaires et tout préférentiellement l'arginine (Arg).
- Xab4 est choisi dans le groupe comprenant les acides aminés hydrophobes ou apolaires, et tout préférentiellement Xab4 est la glycine (Gly).
- rabs est choisi dans le groupe comprenant les acides aminés hydrophobes ou apolaires, les acides aminés polaires larges non chargés, les acides aminés basiques, les acides aminés polaires chargés négativement, les acides aminés polaires petits non chargés, de préférence Xab5 est un acide aminé hydrophobe ou apolaire et tout préférentiellement la phénylalanine (Phe).
- Ca2 et Ca4 sont liés par covalence.
- Xac1 est choisi dans le groupe comprenant les acides aminés basiques, les acides aminés hydrophobes ou apolaires, les acides aminés polaires larges non chargés, de préférence Xac1 est un acide aminé hydrophobe ou apolaire et tout préférentiellement l'alanine (Ala).
- Xac2 est choisi dans le groupe comprenant les acides aminés basiques, les acides aminés hydrophobes ou apolaires, les acides aminés polaires larges non chargés, de préférence Xac2 est un acide aminé basique et tout préférentiellement l'arginine (Arg) ou l'histidine (His).
- Xac3 est choisi dans le groupe comprenant les acides aminés basiques, les acides aminés hydrophobes ou apolaires, les acides aminés polaires petits non chargés, les acides aminés polaires larges non chargés, de préférence Xac3 est un acide aminé hydrophobe ou apolaire et tout préférentiellement la glycine (Gly).
- Xac4 est choisi dans le groupe comprenant les acides aminés basiques, les acides aminés hydrophobes ou apolaires, les acides aminés polaires petits non chargés, de préférence Xac4 est un acide aminé basique et tout préférentiellement l'arginine (Arg).
- Ca3 et Ca6 sont liés par covalence.
- Xad1 est choisi dans le groupe comprenant les acides aminés basiques, les acides aminés hydrophobes ou apolaires, les acides aminés polaires larges non chargés, de préférence Xad1 est un acide aminé basique et tout préférentiellement l'arginine (Arg).
- Xad2 est choisi dans le groupe comprenant les acides aminés hydrophobes ou apolaires, les acides aminés polaires petits non chargés, les acides aminés basiques, de préférence Xad2 est un acide aminé hydrophobe ou apolaire et tout préférentiellement la phénylalanine (Phe).
- Xad3 est choisi dans le groupe comprenant les acides aminés hydrophobes ou apolaires, les acides aminés polaires petits non chargés, les acides aminés basiques, les acides aminés polaires larges non chargés, de préférence un acide aminé hydrophobe ou apolaire, de préférence Xad3 est la proline ou l'hydroxyproline et tout préférentiellement la proline (Pro).
- Xad4 est choisi dans le groupe comprenant les acides aminés hydrophobes ou apolaires, les acides aminés polaires petits non chargés, les acides aminés basiques, les acides aminés polaires larges non chargés, les acides aminés polaires chargés négativement, de préférence Xad4 est un acide aminé polaire petit non chargé et tout préférentiellement la sérine (Ser).
- Xad5 est choisi dans le groupe comprenant les acides aminés hydrophobes ou apolaires, les acides aminés basiques, les acides aminés polaires chargés négativement, de préférence Xad5 est un acide aminé hydrophobe ou apolaire et tout préférentiellement l'isoleucine (Ile).
- Xad6 est choisi dans le groupe comprenant les acides aminés hydrophobes ou apolaires, les acides aminés basiques, les acides aminés polaires larges non chargés, de préférence un acide aminé hydrophobe ou apolaire, de préférence Xad6 est la proline ou l'hydroxyproline et tout préférentiellement la proline (Pro).
- Xad7 est choisi dans le groupe comprenant les acides aminés hydrophobes ou apolaires, les acides aminés polaires larges non chargés, de préférence Xad7 est un acide aminé hydrophobe ou apolaire et tout préférentiellement l'isoleucine (Ile).
- Xad8 est choisi dans le groupe comprenant les acides aminés hydrophobes ou apolaires, de préférence Xad8 est la glycine (Gly).
- Xad9 est choisi dans le groupe comprenant les acides aminés basiques, les acides aminés polaires larges non chargés, les acides aminés polaires petits non chargés, de préférence Xad9 est un acide aminé basique et tout préférentiellement l'arginine (Arg).
- Xae1 est choisi dans le groupe comprenant les acides aminés basiques, les acides aminés hydrophobes ou apolaires, les acides aminés polaires larges non chargés, les acides aminés polaires petits non chargés, de préférence Xae1 est un acide aminé polaire petit non chargé et tout préférentiellement la serine (Ser).
- Xae2 est choisi dans le groupe comprenant les acides aminés basiques, les acides aminés hydrophobes ou apolaires, les acides aminés polaires larges non chargés, les acides aminés polaires petits non chargés, de préférence Xae2 est un acide aminé basique et tout préférentiellement l'arginine (Arg).
- Xae3 est choisi dans le groupe comprenant les acides aminés basiques, les acides aminés hydrophobes ou apolaires, les acides aminés polaires larges non chargés, les acides aminés polaires petits non chargés, les acides aminés polaires chargés négativement, de préférence un acide aminé hydrophobe ou apolaire et tout préférentiellement la phénylalanine (Phe).

Xae4 est choisi dans le groupe comprenant les acides aminés basiques, les acides aminés hydrophobes ou apolaires, les acides aminés polaires larges non chargés, les acides aminés polaires petits non chargés, de préférence Xae4 est un acide aminé hydrophobe ou apolaire et tout préférentiellement la valine (Val).
- Xae5 est choisi dans le groupe comprenant les acides aminés basiques, les acides aminés hydrophobes ou apolaires, les acides aminés polaires larges non chargés, les acides aminés polaires petits non chargés, de préférence Xae5 est un acide aminé polaire large non chargé et tout préférentiellement la glutamine (Gln).
- Xaf1 est choisi dans le groupe comprenant les acides aminés basiques, les acides aminés hydrophobes ou apolaires, de préférence Xaf1 est un acide aminé basique et tout préférentiellement l'arginine (Arg).
- Xaf2 est choisi dans le groupe comprenant les acides aminés basiques, les acides aminés hydrophobes ou apolaires, les acides aminés polaires petits non chargés, de préférence Xaf2 est un acide aminé basique et tout préférentiellement l'arginine (Arg).
- Xaf3 est choisi dans le groupe comprenant les acides aminés basiques, les acides aminés hydrophobes ou apolaires, les acides aminés polaires larges non chargés, de préférence Xaf3 est un acide aminé hydrophobe ou apolaire et tout préférentiellement la valine (Val).
- Xaf4 est choisi dans le groupe comprenant les acides aminés basiques, les acides aminés hydrophobes ou apolaires, de préférence Xaf4 est un acide aminé hydrophobe ou apolaire et tout préférentiellement le tryptophane (Trp).

On entend tout spécialement par :
- acides aminés basiques, la lysine (Lys), l'arginine (Arg), l'histidine (His) ou l'homoarginine ;
- acides aminés polaires chargés négativement, l'acide aspartique (Asp) ou aspartate et l'acide glutamique (Glu) ou glutamate ;
- acides aminés polaires petits non chargés, la sérine (Ser) ou la thréonine (Thr),
- acides aminés polaires larges non chargés, l'asparagine (Asn), la glutamine (Gln) ou la méthionine (Met),
- acides aminés hydrophobes ou apolaires, l'isoleucine (Ile), la leucine (Leu), la phénylalanine (Phe), le tryptophane (Trp), la tyrosine (Tyr), la valine (Val), l'alanine (Ala), la glycine (Gly), la proline (Pro) ou l'hydroxyproline.

L'invention concerne spécifiquement un peptide ou un analogue de sphéniscine qui présente une activité antimicrobienne et comprend trois liaisons intra-moléculaires, et répond à la formule (II) suivante : 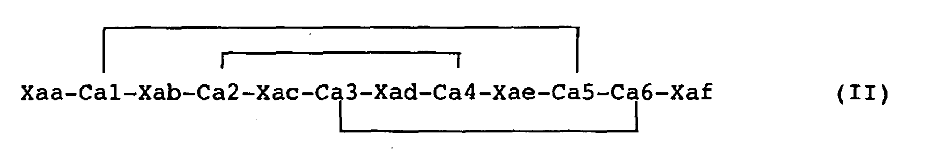 dans laquelle:
Xaa est Ser-Phe-Gly-Leu,
Xab est Arg-Leu-Arg-Arg-Gly-Phe,
Xac est Ala-Xac2 -Gly-Arg,
Xad est Arg-Phe-Pro-Ser-Ile-Pro-Ile-Gly-Arg,
Xae est Ser-Arg-Phe-Val-Gln,
Xaf est Arg-Arg-Val-Trp, et
xac2 est His ou Arg.
de formule (II) suivante :

A titre d'exemple particulier de peptides de formule (II) on peut citer :
- la sphéniscine-1 dont la séquence primaire est la suivante :
   SFGLCRLRRGFCAHGRCRFPSIPIGRCSRFVQCCRRVW (SEQ ID NO. 14)
- la sphéniscine-2 dont la séquence primaire est la suivante :
   SFGLCRLRRGFCARGRCRFPSTPIGRCSRFVQCCRRVW (SEQ ID NO. 15)

Les acides aminés sont généralement représentés par leur code à une lettre, mais ils peuvent être aussi représentés par leur code à trois lettres selon la nomenclature ci-dessous :
A Ala Alanine

| | | |
|---|---|---|
| C | Cys | Cysteine |
| D | Asp | Acide Aspartique |
| E | Glu | Acide Glutamique |
| F | Phe | Phénylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Thréonine |
| V | Val | Valine |
| W | Trp | Tryptophane |
| Y | Tyr | Tyrosine |

Les peptides de l'invention peuvent présenter au niveau de certains des acides aminés des modifications post-traductionnelles naturelles ou chimiques, par exemple le résidu NH,-terminal peut présenter par exemple une acylation, ou le résidu C-terminal peut présenter une modification post-traductionnelle ou chimique, par exemple une amidation, une oxydation ou une estérification. L'invention concerne donc aussi des dérivés des peptides de formules (II), comme ceux dont un ou plusieurs acides aminés sont des acides aminés de conformation D, l'invention couvre également les rétro-peptides et les rétro-inverso-peptides, dès lors qu'ils conservent l'activité antimicrobienne.

L'invention a aussi pour objet l'utilisation des dits peptides dans le domaine agroalimentaire pour protéger des aliments contre les microbes (bactéries et champignons).

L'invention a encore pour objet l'utilisation des dits peptides chez l'homme, l'animal ou les plantes pour prévenir et/ou traiter une infection microbienne, bactérienne et/ou fongique.

La présente invention concerne donc une composition pharmaceutique agronomique ou agroalimentaire comprenant comme agent actif au moins un peptide de l'invention tel que défini précédemment, avantageusement associé dans ladite composition avec un ou plusieurs véhicules, diluant ou excipient pharmaceutiquement acceptables.

Les véhicules, diluants et excipients sont choisis en fonction du type d'application de la composition à titre pharmaceutique, agronomique ou agroalimentaire.

Ainsi, l'invention a encore pour objet l'utilisation d'un peptide de l'invention tel que défini précédemment pour la préparation d'une composition pharmaceutique, agronomique ou agroalimentaire antibactérienne, antibactérienne et/ou antifongique.

Les compositions de l'invention sont utiles tant à titre préventif que curatif.

Pour l'utilisation relative à la conservation des aliments, les peptides de l'invention et les compositions les contenant peuvent se présenter sous la forme de poudre ou de granulés. Ils peuvent être fixés sur les supports contenant les aliments, ou être incorporés directement dans les aliments notamment sous la forme de microorganismes, comme des levures, produisant lesdits peptides.

L'administration de composition pharmaceutique selon l'invention peut être réalisée par l'un quelconque des modes d'administration acceptés pour les agents thérapeutiques, agronomiques ou agroalimentaires. A titre pharmaceutique, chez l'homme ou l'animal, on peut citer l'administration systémique, topique ou encore centrale. L'administration orale peut se faire au moyen de comprimés, gélules, capsules molles, y compris les formulations à libération différée ou prolongée, pilules, poudres, granules, élixirs, teintures, suspensions, sirops et émulsions. L'administration parentérale de composés antibactériens et/ou antifongique se fait généralement par injection intramusculaire ou intraveineuse par perfusion. Les compositions injectables peuvent être préparées sous des formes classiques, soit en suspension ou solution liquide soit sous forme solide convenant à une dissolution extemporanée dans un liquide adéquat, y compris les formulations à libération différée ou prolongée comme l'inclusion des peptides dans des microparticules biodégradables de formulation lipidique, comme des liposomes. D'autres préparations topiques habituelles comprennent les crèmes, les onguents, les lotions, les gels et les sprays aérosols.

L'utilisation des peptides à titre essentiellement préventif consiste à inclure lesdits peptides dans les produits d'hygiène, des pansements, ou encore des litières pour animaux.

En fonction du mode d'administration, les composés peuvent être sous forme solide, semi-solide ou liquide.

Pour les compositions solides, telles que comprimés, pilules, poudres ou granulés à l'état libre ou inclus dans des gélules, ou des microparticules biodégradables de formulation lipidique, comme des liposomes, le principe actif peut être combiné avec des diluants, des lubrifiants, 1 des liants, des absorbants, colorants, aromatisants et édulcorants.

Les compositions selon l'invention peuvent également contenir d'autres substances présentant un intérêt thérapeutique.

Les peptides de l'invention peuvent être administrés sous la forme de doses quotidiennes uniques, ou la posologie quotidienne totale peut être administrée en deux, trois ou quatre doses par jour.

L'invention envisage non seulement les peptides décrits précédemment mais également l'utilisation des séquences polynuclucléotidiques codant ces peptides pour transformer des hôtes, et notamment des cellules animales, végétales ou des procaryotes. Ces séquences sont utilisées conformément aux techniques du génie génétique décrites dans la littérature.

En conséquence, l'invention a également pour objet un polynucléotide codant un peptide décrit précédemment, une molécule d'acide nucléique, ADN ou ARN, comme un vecteur, comprenant ledit polynucléotide, et un hôte, par exemple une cellule animale ou végétale ou encore un procaryote comprenant ladite molécule d'acide nucléique, ainsi que les compositions notamment pharmaceutiques les contenant.

L'invention s'intéresse aussi aux applications agronomiques des peptides décrits précédemment pour rendre des plantes résistantes aux bactéries et champignons phyto-pathogènes et réduire ainsi l'utilisation de pesticides chimiques toxiques pour l'environnement. L'application directe sur la plante d'une quantité efficace de peptides antibactériens et/ou antifongique ou d'une composition en contenant représente une première forme de mise en oeuvre de l'application agronomique. Une deuxième forme de mise en oeuvre de cette application est basée sur des techniques de transgénèse consistant à incorporer de façon stable dans l'ADN d'une cellule végétale, une séquence polynucléotidique codant pour un ou plusieurs peptides ci-dessus. Les cellules végétales ainsi transformées permettent de régénérer une plante transmettant le caractère de résistance aux infections bactériennes et/ou fongique à sa descendance. A titre d'exemples de plantes, on peut citer le riz, le maïs, le colza, la betterave, le blé, le tabac, la tomate, la pomme de terre, etc.

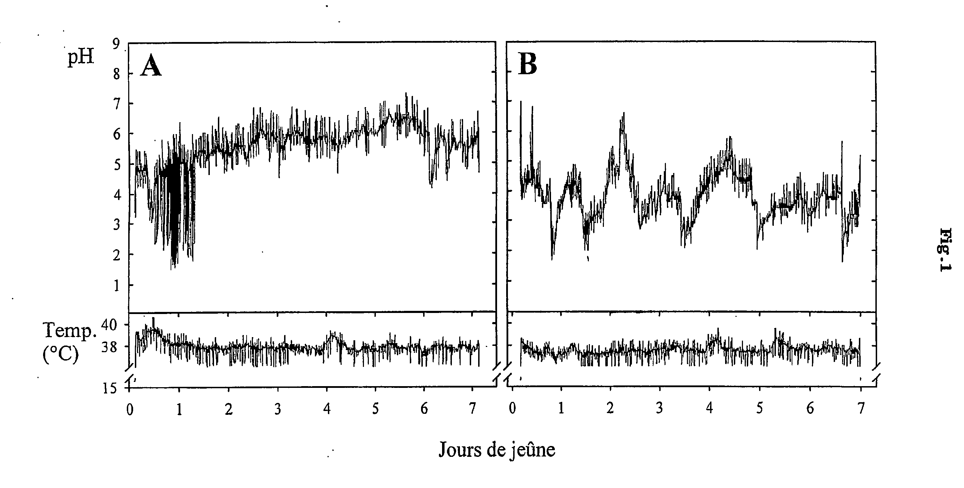

D'autres avantages et caractéristiques de l'invention apparaîtront des exemples qui suivent concernant la mise en évidence, l'isolement et la caractérisation de peptides antimicrobiens à partir d'échantillons de contenu stomacal de mâles de Manchots Royaux ainsi que la forme synthétique de la sphéniscine-2, son spectre d'activité antimicrobienne et l'étude de l'effet du pH sur sa fonctionnalité, et où il sera fait références aux dessins et tableaux en annexe dans desquels
- La figure 1 représente l'évolution du pH gastrique et de la température stomacale chez deux Manchots Royaux mâles pendant la première semaine de leur jeûne d'incubation. Dans un cas, le contenu stomacal est conservé (A), dans l'autre cas, il est digéré (B).

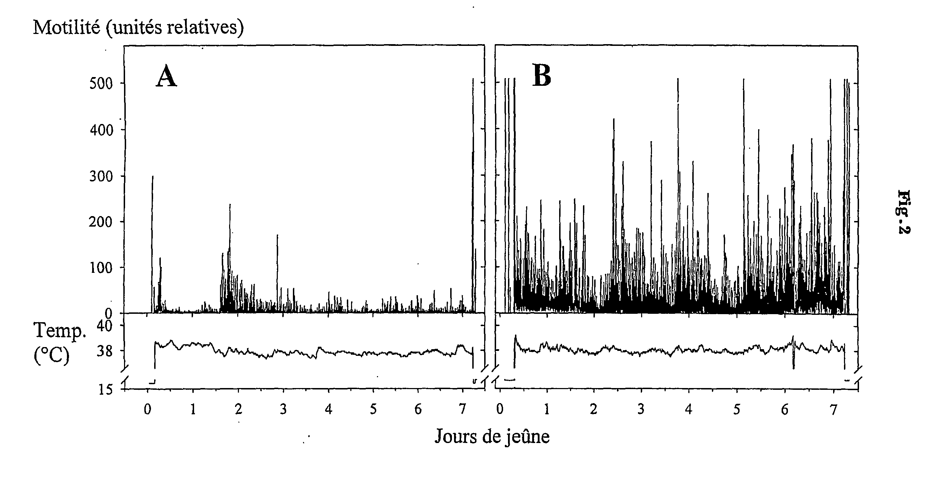

- La figure 2 représente l'évolution de la motilité gastrique et de la température stomacale chez deux Manchots Royaux mâles pendant la première semaine de leur jeûne d'incubation. Dans un cas, le contenu stomacal est conservé (A), dans l'autre cas, il est digéré (B).

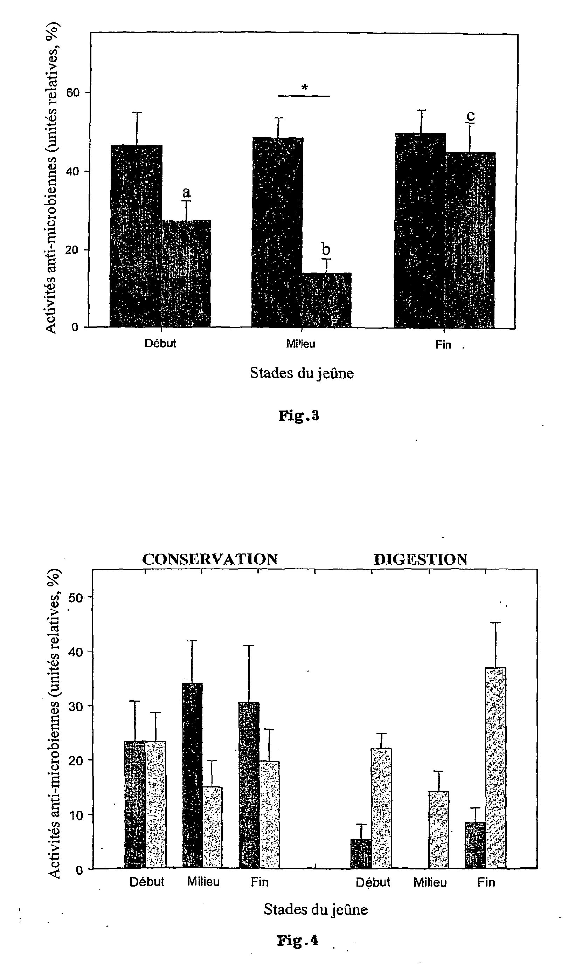

- La figure 3 représente l'évolution des activités antimicrobiennes dans le contenu stomacal de Manchots Royaux à jeun, incubant leur oeuf. Les activités antimicrobiennes ont été testées en déposant des fractions issues d'une première purification par chromatographie des peptides cationiques. Trois souches de microorganismes, représentatives des bactéries Gram - *(Escherichia coli),* Gram + *(Micrococcus luteus)* et des champignons filamenteux *(Neurospora crassa),* ont été utilisées. L'activité antimicrobienne correspond à une diminution ou une absence de croissance des microorganismes pour une fraction testée. Le pourcentage indiqué pour les activités antimicrobiennes correspond à la proportion de fractions ayant ces activités. Deux groupes de Manchots ont été constitués, selon que les oiseaux conservent (histogrammes foncés sur la gauche) ou digèrent (histogrammes plus clairs sur la droite) leur contenu stomacal au cours du jeûne. Lettres : différence au cours du jeûne, pour un même groupe ; *P* < 0,05. Etoile : différence pour un même stade de jeûne, entre les groupes ; *P* < 0,05.
- La figure 4 représente l'évolution des activités antimicrobiennes totales (histogrammes pleins) ou partielles (histogrammes hachurés) dans le contenu stomacal de Manchots Royaux à jeun, incubant leur oeuf. Les activités antimicrobiennes ont été testées en déposant des fractions issues d'une première purification par chromatographie des peptides cationiques. Trois souches de microorganismes, représentatives des bactéries Gram - *(Escherichia coli),* Gram + *(Micrococcus luteus)* et des champignons filamenteux *(Neurospora crassa*), ont été utilisées. S'il n'y a pas de croissance des microorganismes pour une fraction testée, on caractérise l'activité antimicrobienne comme totale. S'il y a seulement une croissance ralentie, il s'agit d'une inhibition partielle. Le pourcentage indiqué pour les activités antimicrobiennes, qu'elles soient totales ou partielles, correspond à la proportion de fractions ayant ces activités. Deux groupes de Manchots ont été constitués, selon que les oiseaux conservent (histogrammes de gauche) ou digèrent (histogrammes à droite) leur contenu stomacal au cours du jeûne.

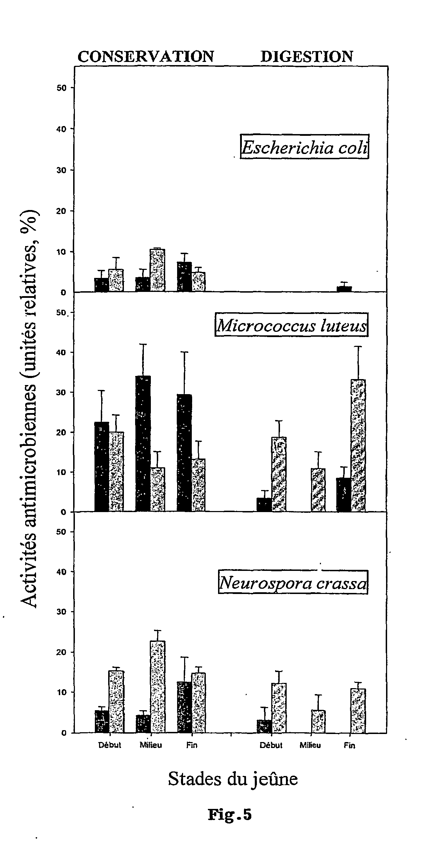

- La figure 5 représente l'évolution des activités antimicrobiennes contre trois types de microorganismes, dans le contenu stomacal de Manchots Royaux à jeun, incubant leur oeuf (Voir la figure 4 pour les légendes).

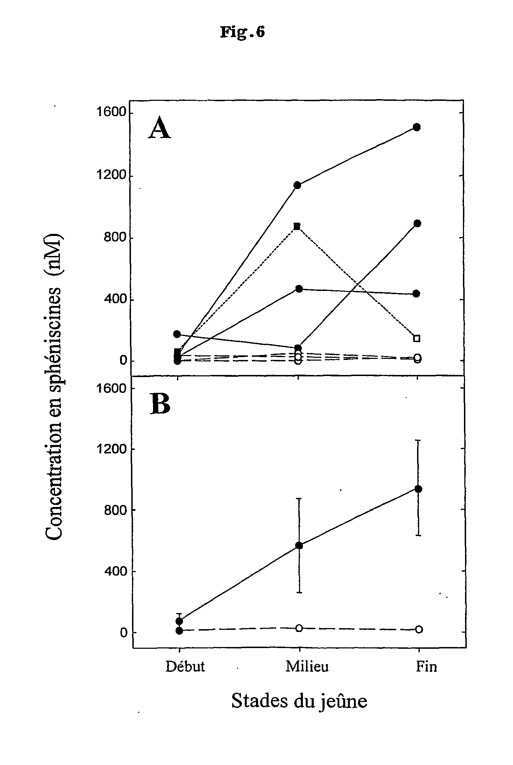

- La figure 6 représente l'évolution quantitative des sphéniscines au cours du jeûne dans le contenu stomacal de Manchots Royaux à jeun, incubant leur oeuf. Deux groupes de Manchots ont été constitués, selon que les oiseaux conservent (ronds noirs et traits pleins) ou digèrent (ronds blancs et pointillés larges) leur contenu stomacal au cours du jeûne. Les valeurs individuelles (A) ou moyennées ± ESM (B) sont représentées. En (A) les valeurs pour un individu ayant conservé son contenu stomacal entre le début et le milieu du jeûne (carrés noirs), puis l'ayant digéré entre le milieu et la fin du jeûne (carrés blancs) sont aussi représentées (pointillés petits).

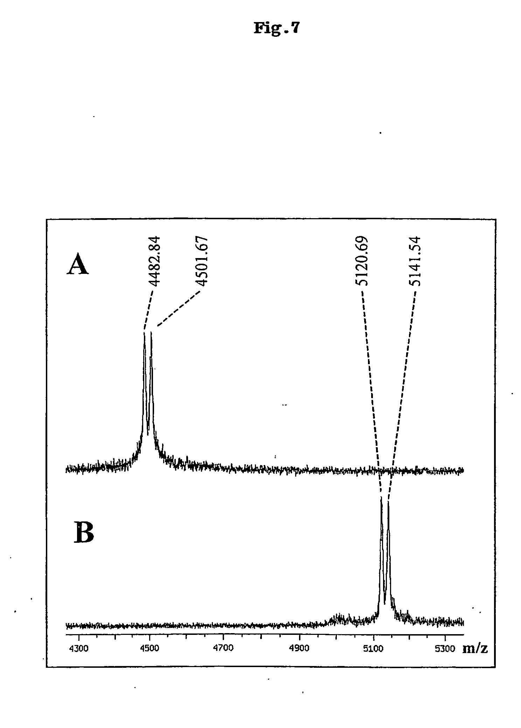

- La figure 7 représente le spectre de masse des sphéniscines, sous forme native (A) ou après réduction et pyridyléthylation des résidus cystéines (B).

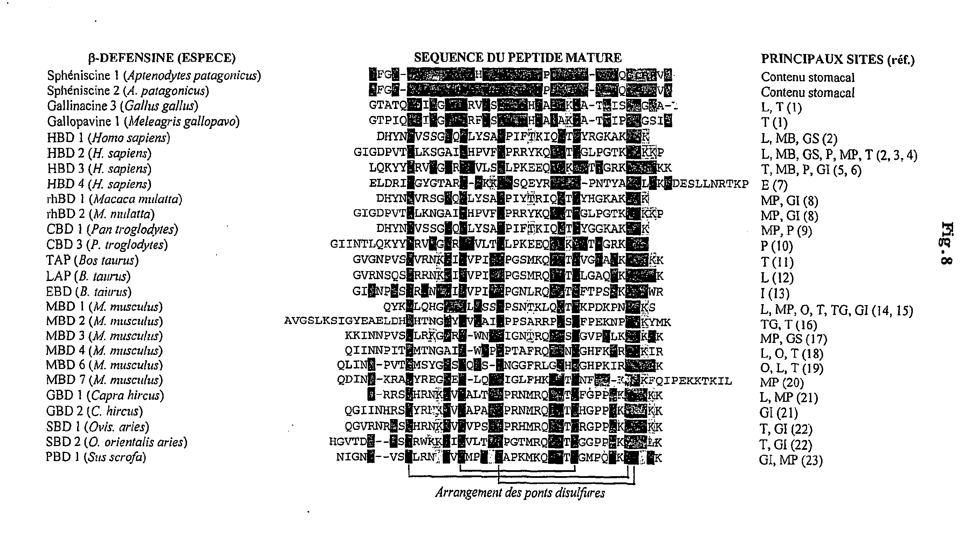

- La figure 8 représente les β-défensines impliquées dans la réponse immunitaire épithéliale chez les Vertébrés. L = langue ; T = trachée ; MB = muqueuse buccale ; GS = glandes salivaires ; P = peau ; Mu muqueuses pulmonaires ; y GI = tractus gastro-intestinal ; E = Estomac ; I = intestin ; 0 = oesophage ; TG = tractus génital. Références : (1) Zhao et al. 2001 ; (2) Harder et al. 1997 ; (3) Hiratsuka et al. 1998) (4) Mathews et al. , 1999 ; (5) Harder et al. 2001 ; (6) Garcia et al. 2001a ; (7) Garcia et al. 2001b ; (8) Bals et al. 2001 ; (9) Duits et al. 2000 ; (10) Duits et al. site AMSDb ; (11) Diamond et al. 1993 ; (12) Schonwetter et al. 1995 ; (13) Tarver et al.1998 ; (14) Bals et al. 1998 ; (15) Huttner et al.1997 ; (16) Morrison et al. 1999 ; (17) Bals et al. 1999 ; (18) Jia et al. 2000 ; (19) Yamaguchi et al. 2001 ; (20) Conejo-Garcia et al. site AMSDb ; (21) Zhao et al. 1999 ; (22) Huttner et al. 1998 ; (23) Zhang et al. 1998.

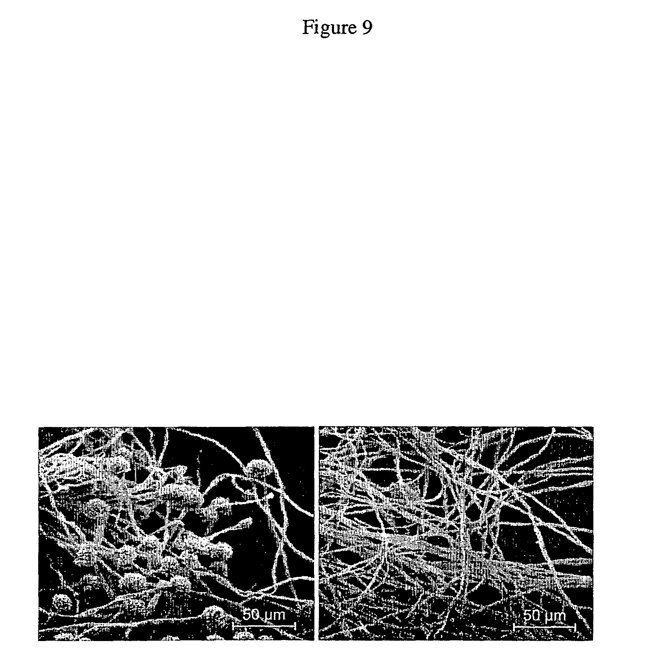

- La figure 9 illustre l'effet délétère de la sphéniscine-2 de synthèse sur la croissance *d'Aspergillus fumigatus,* un champignon filamenteux pathogène (observation au microscope électronique (Philips XL30 LaB6)). (Figure 9A) Pousse contrôle : les têtes aspergillaires terminées par les spores sont bien visibles (x400). (Figure 9B) L'incubation avec 6 µM de sphéniscine-2 entraîne la disparition des têtes aspergillaires. L'effet observé de la sphéniscine-2 sur A. *fumigatus* n'est, par conséquent, pas de type fongicide direct sur les spores, mais se traduit par un arrêt de la croissance par inhibition de la reproduction du champignon puisqu'il n'y a plus formation des têtes aspergillaires, structures impliquées dans le processus de reproduction/prolifération de ce champignon.

### Exemple 1 : Mise en évidence de peptides antimicrobiens dans le contenu stomacal des Manchots Royaux lors du jeûne d'incubation.

Comme la plupart des oiseaux pélagiques, les Manchots Royaux s'alimentent uniquement en mer. Ils alternent des séjours en mer pour se nourrir, avec des périodes de jeûne à terre pour se reproduire et muer. La durée des voyages alimentaires en mer est cependant très variable en durée comme en distance parcourue, en raison d'une variabilité à la fois de la distance à laquelle se trouvent leurs proies, mais aussi de la disponibilité de ces proies au sein même de la zone de prospection alimentaire. Il en résulte que la durée de ces voyages alimentaires peut parfois être doublée (Bost *et al*., 1997).

Pendant la période de reproduction, les deux conjoints se relayent pour assurer l'incubation de l'oeuf unique (54 jours), le mâle assurant normalement la dernière période de l'incubation. Etant donné la variabilité de la durée des voyages en mer, il existe une incertitude sur le sexe du conjoint qui sera sur l'oeuf au moment de l'éclosion. Dans la majeure partie des cas, la femelle revient à temps pour l'éclosion, mais son retour peut être décalé de plus de neuf jours (Gauthier-Clerc *et al*., 2000), alors que les réserves endogènes du poussin nouvellement éclos ne lui assurent que deux ou trois jours d'autonomie.

Il s'avère que le mâle de Manchot Royal a mis au point une adaptation remarquable qui lui permet d'assurer la survie du poussin en cas de retard de la femelle. Il a en effet été montré que le mâle de Manchot Royal est capable, lors de son séjour à terre pour assurer la dernière partie de l'incubation, de garder de la nourriture dans son estomac pendant plus de deux semaines (Gauthier-Clerc *et al.,* 2000). Lui-même jeûne et vit sur ses réserves corporelles. L'état de conservation de cette nourriture est remarquable, sa masse et sa valeur énergétique n'étant pas modifiées (Gauthier-Clerc *et al*., 2002).

Lors de la conservation de cette nourriture, la température stomacale est maintenue à une valeur élevée, en moyenne de 38°C, et le pH gastrique reste entre 5 et 6 (Figure 1). D'autre part, la motricité gastrique est très fortement diminuée en cas de conservation de la nourriture en comparaison avec ce que l'on observe chez un oiseau qui digère son contenu stomacal pendant le jeûne d'incubation (Figure 2). Alors que ces différents paramètres sont favorables à la dégradation de la nourriture par les bactéries présentes dans le contenu, l'analyse qualitative et quantitative de cette nourriture a montré qu'il n'en était rien.

Une étude bactériologique a permis de montrer qu'il existe une très forte proportion de bactéries viables dans le contenu stomacal de Manchots qui le conservent efficacement au cours du jeûne, mais elles ne peuvent apparemment pas se développer. Ces bactéries présentent en effet les caractéristiques morphologiques de bactéries placées en condition de stress environnemental. D'autre part, les bactéries cultivables sont principalement des bactéries environnementales telles que des *Corynebacterium, Moraxella, Staphylococcus, Micrococcus* et *Streptococcus* spp. ainsi que des bactéries très vraisemblablement issues des proies, à savoir *Clostridium* spp. Par contre, plusieurs bactéries de type *Pseudomonas* et *Vibrio* spp. présentes dans le milieu marin (Mac Cormack & Fraile, 1990) n'ont pas été détectées. De même aucune bactérie entérique n'a été mise en évidence bien que de telles bactéries aient été observées chez le Manchot Royal (Soucek & Mushin, 1970). Les résultats de l'analyse de la flore bactérienne sont eux aussi en faveur d'une protection de la nourriture stockée contre la dégradation par les bactéries. Un contrôle de la flore bactérienne par des substances antimicrobiennes, produites par les oiseaux, pourrait expliquer cette conservation.

La présence de peptides antimicrobiens a été recherchée dans le contenu stomacal des Manchots Royaux lors du jeûne d'incubation. Il a ainsi été réalisé :
- un suivi individuel au cours du jeûne des peptides antimicrobiens présents dans le contenu stomacal. Plusieurs prélèvements ont été réalisés pour un même individu au cours du jeûne,
- une comparaison entre deux groupes d'oiseaux, selon que ces derniers conservaient (groupe « conservation ») ou digéraient (groupe « digestion ») leur contenu stomacal au cours du jeûne (voir l'exemple 2.1. pour le protocole détaillé).

Quatre souches de microorganismes tests ont été sélectionnées pour cribler la présence de peptides antimicrobiens dans des extraits de contenu stomacal : *Micrococcus luteus* (Gram positif), *Escherichia coli* SBS 363 (Gram négatif), *Neurospora crassa* (champignon filamenteux), et la levure *Candida albicans.* Ces souches ont été sélectionnées en raison de leur sensibilité élevée pour les peptides antimicrobiens (voir les exemples 2.2, 2.3 et 2.4. pour le détail du protocole). Nous restreindrons notre description aux activités dirigées contre les souches de bactéries *E. coli, M. luteus,* et *N. crassa.*

Trois résultats majeurs se dégagent :
- Il existe des molécules à activité antimicrobienne dans les échantillons de contenu stomacal de mâle de Manchots Royaux.
- Ces activités antimicrobiennes s'expriment différemment chez les individus du groupe « conservation », comparativement aux individus du groupe « digestion ». Ces différences se situent au niveau quantitatif et qualitatif.

Au niveau quantitatif :
- Les activités inhibitrices des microorganismes sont plus importantes dans le groupe « conservation » comparativement au groupe « digestion », en début et milieu de jeûne (Figure 3). La distinction entre les activités inhibitrices totales et partielles montre que la différence entre les deux groupes est liée à une plus grande proportion d'activités inhibitrices totales dans le groupe « conservation » comparativement au groupe « digestion », ceci tout au long du jeûne (Figure 4).
- Les activités inhibitrices (totales et partielles) sont maintenues tout au long du jeûne dans le groupe « conservation ». Une très forte diminution en milieu de jeûne est observée dans le groupe « digestion ».

Au niveau qualitatif (Figure 5) :
- Pour les trois types de microorganismes testés, les activités inhibitrices sont plus importantes dans le groupe « conservation » comparativement au groupe « digestion », ceci tout au long du jeûne. Ces activités sont essentiellement dirigées contre la bactérie Gram + testée (*M*. *luteus).*
- A une exception, seuls les échantillons des individus du groupe « conservation » présentent des activités inhibitrices dirigées contre la bactérie Gram - testée (*E. coli).*

En conclusion, ces résultats sont fortement en faveur de l'implication de substances à activité antimicrobienne dans le phénomène de conservation du bol alimentaire lors du jeûne d'incubation chez le mâle de Manchot Royal.

### Exemple 2 Isolement et caractérisation de peptides antimicrobiens à partir d'échantillons de contenu stomacal de mâles de Manchots Royaux.

### 2.1 Oiseaux et prélèvement de contenu stomacal.

Tous les échantillons de contenu stomacal ont été prélevés sur des Manchots Royaux mâles, dans leur milieu naturel. La colonie de Manchots se trouve sur l'Ile de la Possession (46°25'S - 51°45'E), dans l'Archipel des Iles Crozet. Les prélèvements ont été effectués au cours de la période d'incubation, à l'aide d'une sonde gastrique. Cette étude, menée dans une zone protégée, et sur une espèce protégée, 1 a été réalisée avec l'autorisation du Territoire des Terres Australes et Antarctiques Françaises (Décision n°2000-59 du 16 Octobre 2000) et l'autorisation du Ministère de l'Aménagement du Territoire et de l'Environnement (autorisation 00/240/AUT du 30 Août 2000).

a) Oiseaux : Les mâles de Manchot Royal ont été identifiés en début de la période d'incubation, juste après la ponte et le passage de l'oeuf entre la femelle et le mâle, par une bague plastique placée temporairement au niveau de l'aileron.

Les prélèvements de contenu stomacal ont été réalisés pendant la dernière période de l'incubation, assurée par le mâle. Afin de réaliser un suivi individuel du contenu stomacal, les prélèvements ont, pour un même oiseau, été effectués à trois stades différents du jeûne : le début, le milieu du jeûne (environ 7 jours) et la fin du jeûne. Pour les prélèvements du début et du milieu de jeûne, l'oiseau a été étudié alors qu'il était sur son oeuf. Pour le stade de fin de jeûne, le mâle a été capturé juste après la relève par la femelle, en dehors de la colonie.

Les prélèvements ont été effectués au calme, en dehors de la colonie. Afin de limiter le stress de l'oiseau, celui-ci a été placé à l'obscurité grâce à une cagoule. De même, l'oeuf a été provisoirement remplacé par un oeuf en plâtre, préalablement réchauffé. L'oeuf naturel a été placé en couveuse le temps de la manipulation. Après manipulation, les oiseaux ont été reposés à l'endroit exact de la capture. Tous les oiseaux étudiés ont mené à terme l'incubation.

Deux groupes d'oiseaux ont été constitués, selon que les oiseaux conservaient (groupe « conservation », n = 3), ou digéraient (groupe «digestion », n = 3) leur contenu stomacal au cours du jeûne d'incubation.

b) Prélèvement des échantillons et conditionnement : Les échantillons de contenu stomacal ont été prélevés par une méthode non-destructive et non-invasive d'intubation et d'aspiration. Afin d'obtenir un échantillonnage homogène une quantité supérieure à celle nécessaire a été prélevée en plusieurs fois, puis immédiatement homogénéisée dans un récipient maintenu dans de la glace. C'est seulement après cette homogénéisation, que l'échantillonnage a été réalisé. Les échantillons ont été conservés à -80°C. Lors du rapatriement des échantillons jusqu'en France métropolitaine, ceux-ci ont été maintenus congelés successivement à -80°C puis dans de la carboglace selon la technique d'acheminement (bateau puis avion), avant d'être à nouveau placé à -80°C jusqu'à leur analyse avec un respect strict de la chaîne du froid.

### 2.2 Extraction des peptides cationiques hydrophobes et prépurification.

L'extraction des peptides cationiques hydrophobes a été réalisée à partir des échantillons de contenu stomacal congelés. Le contenu stomacal a été broyé à l'aide d'un ultraturax puis d'une sonde à ultrasons, dans le milieu d'extraction composé d'acide trifluoroacétique (TFA, 0,2%) contenant de l'aprotinine (22,5 µg/ml concentration finale) comme inhibiteur de protéase. Les échantillons ont été maintenus au froid, dans de la glace, pendant toute l'étape de broyage. Le rapport volume d'échantillon / volume de milieu d'extraction a été sélectionné de 1/10 à la fin du broyage. Les peptides ont été extraits à pH 2,5-3 sous agitation durant une nuit en chambre froide. Après centrifugation (10000 rpm durant 10 min à 6°C), le surnageant a été prépurifié par extraction en phase solide sur des cartouches de phase inverse Sep-Pak C₁₈. Vac (5g de phase, Waters™). La cartouche a été solvatée au méthanol et équilibrée avec de l'eau acidifiée (0,05% TFA). L'élution des peptides/polypeptides et protéines a été réalisée avec une solution de 80% d'acétonitrile dans de l'eau acidifiée. La fraction éluée a été lyophilisée avant purification par chromatographie liquide à haute performance (CLHP).

### 2.3 Purification des peptides cationiques.

### a) Etape 1 : Purification sur colonne semi-préparative.

La fraction 80% Sep-Pak a été soumise à une chromatographie sur support de phase inverse sur une colonne semi-préparative Aquapore RP-300 C₁₈ (250 x 7 mm, Brownlee™), équilibrée avec une solution d'acétonitrile à 2% dans de l'eau acidifiée. Les fractions ont été séparées à l'aide d'un gradient linéaire de 2% à 72% d'acétonitrile dans de l'eau acidifiée en 70 min avec un débit de 1,3 ml/min.

Cette étape de purification a été réalisée sous température contrôlée (20-22°C) sur un système Beckman Gold HPLC équipé d'un détecteur Beckman 168 photoarray. Les molécules éluées de la colonne ont été détectées par leur absorbance à 225 nm.

Les différentes fractions ont été collectées manuellement, séchées sous vide (Speed-Vac, Savant) et reconstituées dans 150 µl d'eau ultrapure (Millipore™) préalablement filtrée (Millipore™), avant analyse de l'activité antimicrobienne.

### b) Etape 2 Purification sur colonne analytique.

La purification des molécules à activité antimicrobienne (activité totale dirigée contre les deux souches de bactéries et la souche de champignons filamenteux) a été réalisée à partir d'une fraction issue du contenu stomacal prélevé sur un oiseau appartenant au groupe « conservation » en fin de jeûne.

La seconde étape de purification a été réalisée sur une colonne analytique Aquapore OD-300 (220 x 4,6 mm, Brownlee™). L'élution a été réalisée à l'aide d'un gradient biphasique d'acétonitrile dans l'eau acidifiée, de 2 à 23% en 10 min puis de 23 à 38% en 45 min avec un débit de 0,8 ml/min. Les différentes fractions ont été collectées manuellement, séchées sous vide (Speed-Vac, Savant) et reconstituées dans 70 µl d'eau MilliQ (Millipore™). Afin de limiter l'utilisation du produit actif pour les tests biologiques, seule la souche à Gram négatif *E. coli* a été testée. Les fractions inactives ont alors été testées sur N. *crassa.* Aucune activité n'a été détectée, suggérant que l'activité dirigée contre le champignon filamenteux N. *crassa,* enregistrée au cours de l'étape de purification précédente, est bien liée à la fraction présentant l'activité dirigée contre la bactérie à Gram négatif *E. coli.*

### c) Etape 3 : Phase finale de purification sur colonne analytique.

La troisième étape de purification a été réalisée sur la même colonne analytique que dans l'étape 2 en utilisant comme conditions d'élution un gradient biphasique d'acétonitrile dans l'eau acidifiée, de 2 à 20% en 10 min et de 20 à 30% en 50 min avec un débit de 0,8 ml/min. Les fractions ont été recueillies, séchées sous vide, reprise dans 40 µl d'eau et analysées pour leur activité antibactérienne contre *E. coli.*

Ces deux dernières étapes de purification ont été réalisées sous température contrôlée, sur un système CLHP biocompatible (all PEEK Waters, Modèle Waters 626) relié à un détecteur à absorbance variable (Waters 486). Les molécules éluées de la colonne ont été détectées par leur absorbance à 225 nm, et l'activité antimicrobienne mesurée suivant la procédure décrite dans l'Exemple 2.4 ci-dessous.

### 2.4 Détection des activités antimicrobiennes.

L'activité antimicrobienne a été mesurée sur quatre souches de microorganismes : *Micrococcus* luteus (Gram positif; collection de l'Institut Pasteur, Paris), *Escherichia coli* SBS 363 (Gram négatif; don de M. Boquet du Centre d'Etudes Nucléaires de Saclay), *Neurospora crassa* (champignon filamenteux; mycothèque de la Société Clause, Paris) et *Candida albicans* (levure; don du Dr. Koenig, Hôpital Civil, Strasbourg). Ces quatre souches ont été sélectionnées dans des collections publiques ou privées en raison de leur sensibilité reconnue à des peptides antimicrobiens naturels.

Les bactéries à tester ont été mises en suspension dans un milieu nutritif approprié de type « Poor Broth », correspondant à une solution de bactotryptone à 10 g/l additionnée de NaCl 5 g/l préparée dans de l'eau ultrapure.

Les spores du champignon *N. crassa* à tester ont été mises en suspension dans un milieu de culture approprié de type « infusion de Pomme de terre - Glucose ». De préférence on utilise 12 g de milieu Potato Dextrose Broth (1/2PDB, Difco) pour 1 1 d'eau déminéralisée. Deux antibiotiques ont été rajoutés au milieu de culture : la céfotaxine (concentration finale à 100 µg/ml) et la tétracycline (concentration finale à 10 µg/ml).

La souche de levure *C*. *albicans* à tester a été mise en incubation dans un milieu de culture approprié de type « Sabouraud ».

Les activités antimicrobiennes ont été détectées par un test d'inhibition de croissance en milieu liquide dans des plaques de microtitration (Hétru & Bulet, 1997). On dépose 10 µl de chaque fraction collectée dans des plaques de microtitration en présence de 90 µl de milieu de culture contenant les microorganismes (à une concentration finale équivalente à 1 mDO à 600 nm).

Pour les bactéries et la levure, l'incubation a été réalisée à 30°C pendant 12-24 h sous agitation. La croissance des microorganismes a été mesurée en suivant l'absorbance à 600 nm à l'aide d'un spectrophotomètre lecteur de plaque de microtitration.

Pour les champignons filamenteux, l'incubation a été réalisée à 37°C sous atmosphère humide pendant 48 heures. La croissance fongique a été observée au microscope photonique après 24 h et quantifiée après 48 h par mesure de l'absorbance à 600 nm à l'aide d'un spectrophotomètre lecteur de plaque de microtitration.

### 2.5 Caractérisation structurale des sphéniscines.

### 2.5.1 Analyse par spectrométrie de masse.

La pureté et la détermination de la masse moléculaire des molécules bioactives ont été réalisées par spectrométrie de masse selon la technique dite de mesure du temps de vol après désorption ionisation laser assistée par matrice (Technique MALDI-TOF). Le matériel utilisé est un spectromètre de masse Brucker BIFLEX- III (Bremen, Allemagne) équipé d'une optique haute résolution SCOUT™ et d'un réflectron. Cet instrument a un potentiel maximum d'accélération de 20kV et peut être utilisé soit en mode linéaire soit en mode réflectron. L'ionisation est réalisée avec un faisceau à 337 nm émis par un laser à azote à une fréquence de 3Hz. Les spectres de masse ont été calibrés de façon externe avec un mélange standard de peptides de la drosophile à savoir la drosocine, la metchnikowine et la drosomycine de masse moléculaire connue, respectivement à 2199,5 Da, 3046,4 Da et 4890,5 Da (Bullet, 1999).

L'échantillon à analyser a été préparé selon la technique du sandwich (Kussmann et. al., 1997) exposée comme suit : 0,5µl d'échantillon ont été déposés sur une fine couche de cristaux d'acide α-cyano-4-hydroxycinnamique (4-HCCA, Sigma) obtenue par une évaporation rapide d'une solution saturée dans l'acétone. Le tout a été recouvert par 0,5 µl de matrice 4-HCCA à saturation dans une solution d'acétonitrile 50% dans de l'eau. Après séchage sous un léger vide, l'échantillon a été lavé par 1,5 µl de TFA 0,1% avant d'être séché à nouveau et introduit dans le spectromètre de masse.

Le spectre de masse obtenu pour la fraction isolée lors de l'étape 3 de l'exemple 2.3 ci-dessus est présenté sur la figure 7A. Cette fraction contenait deux molécules de masse moléculaire en MH⁺ à 4482,84 et 4501,67. L'étape suivante a été de vérifier la présence de résidus cystéines dans les molécules antimicrobiennes isolées et éventuellement de modifier ces résidus cystéines pour favoriser l'identification de la séquence au cours du séquençage peptidique par dégradation d'Edman.

### 2.5.2 Vérification de la présence de résidus cystéines et détermination de leur nombre réduction et S-pyridyléthylation.

### a) Etape 1 : Traitement chimique.

La présence de résidus cystéine a été déterminée après réduction et S-pyridyléthylation. Une fraction aliquote des peptides purifiés à l'étape 3 de l'exemple 2.3 a été soumise à une étape de réduction chimique avec 4 µl de dithiothréitol (concentration finale 2,2 M) dans 40 µl de tampon Tris/HCl 0,5 M, pH 8,3 contenant 2 mM d'EDTA et 6 M de chlorure de guanidium. Le milieu réactionnel a été placé sous atmosphère d'azote. Après 60 min d'incubation à l'obscurité, 2 µl de 4-vinyl pyridine (agent alkylant) ont été ajoutés à la réaction. Le milieu réactionnel a été placé sous atmosphère d'azote et incubée 10 min et à 45°C à l'obscurité. La réaction a été arrêtée par acidification avec 50 µl de TFA 10%.

### b) Etape 2 : Purification des peptides réduits et alkylés.

Les peptides pyridyléthylés ont été purifiés du milieu réactionnel par chromatographie de phase inverse en utilisant une colonne narrow bore C₁₈ (DeltaPak HPIC₁₈, 2 x 150 mm, Waters™). L'élution a été réalisée à l'aide d'un gradient linéaire d'acétonitrile dans l'eau acidifiée de 2 à 60% en 90 min avec un débit de 0,2 ml/min. Cette séparation a été réalisée sous température contrôlée sur un système CLHP biocompatible (all PEEK Waters, Modèle Waters 626) relié à un détecteur à absorbance variable (Waters 486). Les molécules éluées de la colonne ont été détectées par leur absorbance à 225 nm.

### c) Etape 3 : Détermination du nombre de résidus cystéines par spectrométrie de masse.

La mesure de masse des peptides pyridyléthylés a été effectuée par un spectromètre de masse MALDI-TOF selon le protocole énoncé dans l'exemple 2.5.1. Cette fraction contenait deux molécules de masse moléculaire en MH⁺ à 5120,69 et 5141,54 (Figure 7B). La différence de masse moléculaire mesurée avant et après réduction pour les deux molécules correspond à l'ajout de 6 résidus 4-vinyl pyridine (6 x 106 Da). Ceci confirme la présence de 6 résidus cystéines dans la molécule à 4482.84 (m/z) et dans celle à 4501.67 (m/z) et leur implication dans la formation de trois ponts disulfure intramoléculaires.

### 2.5.3 Détermination des séquences primaires des sphéniscines.

### a) Etape 1 : Séquençage par dégradation d'Edman.

Le séquençage automatique par dégradation d'Edman des peptides S-pyridyléthylés a été réalisé sur un séquenceur ABI473A (Applied Biosystems Inc.). Deux séquences primaires de 38 acides aminés, ne différant que par un acide aminé en position 14 (histidine versus arginine), ont été obtenues avec une ambiguïté sur les acides aminés en position 31 et 37. Afin de lever cette ambiguïté, les deux peptides ont été traités à la chymotrypsine.

### b) Etape 2 : Digestion à la chymotrypsine et analyse des fragments chymotrypsiques par spectrométrie de masse.

La fraction (4 µl) contenant les peptides S-pyridyléthylés ont été mis en solution dans 20 µl de tampon Tris 100 mM à pH 7,8, contenant 10 mM de CaCl₂, en présence de chymotrypsine au ratio de 1/20 (enzyme/peptides, poids/poids). Des fractions aliquotes (0,5 µl) ont été analysées par spectrométrie de masse MALDI-TOF après 30 min et 55 min d'incubation à 30°C. Les échantillons ont été analysés par spectrométrie de masse MALDI-TOF selon le protocole décrit dans l'exemple 1.5.1. Parmi les fragments chymotrypsiques observés après 55 min, un des fragments à la masse moléculaire mesurée à 1261,05 (m/z) a permis de confirmer et de lever les ambiguïtés observées après le séquençage par dégradation d'Edman. Séquences C-terminales identiques pour les deux peptides.

L'ensemble des techniques utilisées a ainsi permis d'obtenir la structure complète de la sphéniscine-1 et de la sphéniscine-2.

La séquence primaire de la sphéniscine-1 est la suivante :
SFGLCRLRRGFCAHGRCRFPSIPIGRCSRFVQCCRRVW (SEQ ID NO. 14)
Masse moléculaire calculée 4483,38 MH⁺
Masse moléculaire mesurée : 4482,84 MH⁺

La séquence primaire de la sphéniscine-2 est la suivante :
SFGLCRLRRGFCARGRCRFPSIPIGRCSRFVQCCRRVW (SEQ ID NO. 15)
Masse moléculaire calculée : 4502,42 MH⁺
Masse moléculaire mesurée : 4501,67 MH⁺

L'analyse dans les banques de données protéiques et nucléotidiques (FASTA Genome, NCBI-TBLASTN) a permis de montrer que les sphéniscines appartiennent à la famille des β-défensines, peptides antimicrobiens largement répandus dans le monde animal. Une comparaison détaillée des séquences des sphéniscines a été réalisée avec les séquences des β-défensines connues trouvées dans les épithélia de vertébrés et en l'occurrence chez la poule *(Gallus gallus)* et la pintade *(Meleagris gallopavo).* Les homologies observées (Figure 8) montrent clairement que les sphéniscines, isolées à partir d'un contenu stomacal de Manchot Royal, sont bien produites et sécrétées par l'oiseau et ne proviennent pas de l'alimentation elle-même.

### Exemple 3 : Evolution des sphéniscines dans le contenu stomacal au cours du jeûne d'incubation chez le Manchot Royal.

### 3.1 Détection des sphéniscines dans le contenu stomacal par spectrométrie de masse MALDI-TOF.

Les analyses sont réalisées pour chaque échantillon de contenu stomacal, sur les fractions issues de la première étape de purification (cf. Exemple 2.3 Etape 1). La procédure d'analyse par spectrométrie de masse MALDI-TOF est celle décrite dans l'Exemple 2.5.1.

Toutes les fractions issues de la première étape de purification provenant d'un même individu dans un stade de jeûne donné et contenant de la sphéniscine 1 ou/et 2 ont été regroupées. Les sphéniscines ont été purifiées par chromatographies successives sur colonnes de phase inverse appropriées en utilisant des gradients d'acétonitrile adaptés selon le modèle décrit ci-dessus dans l'Exemple 2.3.

Les étapes de purification ont été réalisées sous température contrôlée, sur un système CLHP biocompatible (all PEEK Waters, Modèle Waters 626) relié à un détecteur à absorbance variable (Waters 486). Les molécules éluées de la colonne ont été détectées par leur absorbance à 225 nm et la présence des sphéniscines 1 et 2 confirmée par spectrométrie de masse MALDI-TOF.

### 3.2 Quantification et évolution des sphéniscines dans le contenu stomacal au cours du jeûne d'incubation chez le Manchot Royal.

### a) Etape 1 : Analyse par électrophorèse capillaire de zone.

La première étape permettant la quantification des sphéniscines a consisté à réaliser une électrophorèse capillaire de zone sur un modèle 270 A-HT (Applied Biosystems Inc.). Quatre nl de chaque "pool" de fractions (12 µl) contenant de la sphéniscine (déterminée par spectrométrie de masse MALDI-TOF) ont été injectés sous assistance par le vide dans un capillaire de silice (72 cm x 50 µm). L'analyse a été réalisée en tampon citrate 20 mM à pH 2,5. L'électrophorèse a été réalisée à 20 kV de l'anode à la cathode pendant 20 min à 30°C. La migration a été enregistrée à 200 nm. Pour chaque échantillon, la surface du pic correspondant aux sphéniscines 1 et 2 a été déterminée.

### b) Etape 2 : Quantification.

De manière à pouvoir effectuer la quantification, l'aire du pic obtenu par électrophorèse capillaire a été comparée à celle d'une solution calibrée de sphéniscines. Cette solution correspond à l'échantillon analysé par dégradation d'Edman. La quantité précise de sphéniscines séquencée a été déterminée par mesure du rendement répétitif et du rendement initial obtenu durant le séquençage par dégradation d'Edman.

Les quantités observées ont alors été ramenées aux concentrations présentes dans l'échantillon initial du contenu stomacal. Les données obtenues montrent clairement une fluctuation importante de la teneur en sphéniscines du contenu stomacal entre les deux groupes traités : le groupe "conservation" et "digestion" (Figure 6). D'autre part, dans le groupe "conservation", une augmentation nette du taux de sphéniscines est observée entre le début et la fin du jeûne.

### Exemple 4 : Forme synthétique de la sphéniscine-2, son spectre d'activité antimicrobienne et l'étude de l'effet du pH sur sa fonctionnalité.

### 4.1. Objet de ces études et principaux résultats.

Dans le cadre de la présente invention, de nouvelles études ont été réalisées afin d'évaluer les propriétés antimicrobiennes ainsi que la structure secondaire (tri-dimensionnelle, 3D) des peptides (sphéniscines) objet de ladite invention. Dans le but d'effectuer ces études, un peptide de synthèse de composition et de structure identiques à l'un des variants des peptides de l'invention qu'est la sphéniscine-2 a été produit chimiquement. Grâce à la sphéniscine-2 synthétique, il a pu être entrepris l'étude:
- du spectre d'activité de la sphéniscine-2 sur une large gamme de microorganismes, dont des souches pathogènes pour l'Homme. Des souches de bactéries à Gram-positif et à Gram-négatif, de levures et de champignons filamenteux ont été utilisées.
- de l'activité de la sphéniscine-2 en fonction du pH du milieu. En effet, dans le cas de la conservation du bol alimentaire chez le Manchot Royal il a été montré que le pH oscillait entre 4 et 6 (Thouzeau et al., 2003). Il s'agissait de tester, *in vitro,* si un tel pH pouvait avoir une incidence sur l'activité de la sphéniscine *in vivo.* Cette étude était essentielle dans le contexte de l'utilisation potentielle de ces molécules pour la conservation alimentaire ou la lutte contre des infections microbiennes en milieu gastro-intestinal.

Cinq résultats majeurs se dégagent de ces deux études :
- La sphéniscine-2 affecte fortement la pousse d'un nombre important de souches de bactéries à Gram-positif (Tableau 1). Cet effet est principalement de type bactéricide (lyse des cellules bactériennes);
- La sphéniscine-2 est également active contre une grande variété de souches de bactéries à Gram-négatif (Tableau 1). Cet effet, contrairement à ce qui est observé contre les germes à Gram-positif, est surtout de type bactériostatique (arrêt de la multiplication bactérienne);
- La sphéniscine-2 possède une activité antifongique aussi bien contre les levures que les champignons filamenteux (Tableau 1). Cet effet peut être fongicide (lyse des spores) mais également affecter la phase de reproduction des champignons par blocage de la sporulation comme nous avons pu l'observer sur le champignon pathogène de l'Homme, *Aspergillus fumigatus.* Ce dernier effet est illustré par la figure 9 en annexe ;
- L'existence de modes d'action de la sphéniscine différents suivant les souches de microorganismes affectés par ce peptide ;
- La sphéniscine-2 est toujours fonctionnelle dans la gamme de pH observée dans le contenu stomacal des Manchots, soit de pH 4 à pH 6 (Tableau 2).

En conclusion, la mise en évidence du large spectre d'activité de la sphéniscine-2, affectant la croissance de bactéries et de champignons pathogènes de plantes et de l'Homme, et le maintien de la fonctionnalité de ce peptide aux pH observés *in vivo* dans l'estomac sont des arguments supplémentaires en faveur de l'implication de substances à activité antimicrobienne dans le phénomène de conservation du bol alimentaire lors du jeûne d'incubation chez le Manchot Royal. Ces propriétés biologiques sont également en faveur de l'utilisation de ces molécules pour la lutte antimicrobienne dans un environnement dans lequel le pH serait modérément acide.

Les sous-paragraphes qui suivent apportent des caractéristiques supplémentaires concernant la forme synthétique de la sphéniscine-2, son spectre d'activité antimicrobienne et l'étude de l'effet du pH sur sa fonctionnalité.

### 4.2. Obtention de la forme synthétique de la sphéniscine-2.

L'étude des propriétés de la sphéniscine-2 nécessitait de disposer d'une quantité suffisante de peptide, ce qu'il était difficile d'obtenir à partir d'extrait de contenu stomacal de Manchot. Une forme synthétique de la sphéniscine-2 a été obtenue auprès du Laboratoire ALTERGEN (Schiltigheim, France). La sphénisnine-2 a été produite pas synthèse chimique suivant une procédure connue de l'homme du métier. Après renaturation de la molécule dans des conditions adaptées connues de l'homme de l'art, la pureté de la molécule et son identité avec la molécule native ont été contrôlées en utilisant la procédure décrite ci-dessous :
a) Traitement du brut de synthèse La poudre constituant le brut de synthèse a été lavée des reliquats d'acide trifluoroacétique présents par plusieurs lavages successifs à l'aide d'une solution d'ACN 50% ; lavages entrecoupés d'une étape de séchage sous vide (Speed-Vac, Savant) suivi d'une étape de lyophilisation.
b) Vérification de la masse moléculaire et de l'organisation des ponts disulfures : L'identité du produit synthétique ainsi lavé a été vérifiée et comparée avec celle de la molécule native, issue du contenu stomacal. La masse moléculaire ainsi que la pureté de la sphéniscine de synthèse ont été vérifiées par spectrométrie de masse selon la technique dite de mesure du temps de vol après désorption ionisation laser assistée par matrice (Technique MALDI-TOF, pour les détails de la technique voir l'exemple 2.5.1.). L'agencement des ponts disulfures, caractéristique de la molécule (Cysl-Cys5, Cys2-Cys4, Cys3-Cys6), a été vérifié par clivage enzymatique à la trypsine (Boehringer Mannheim, Allemagne) suivi d'une empreinte massique par MALDI-TOF des fragments issus de cette digestion.

L'ensemble des résultats obtenus a montré que la sphéniscine de synthèse était totalement identique à la molécule sphéniscine-2 naturelle aussi bien au niveau de sa structure primaire que par son arrangement des ponts disulfures :
Sphéniscine-2 naturelle (Sphe-2N) et synthétique (Sphe-2S):
Masse moléculaire calculée : 4502,42 MH⁺
Masse moléculaire mesurée (Sphe-2N): 4501,67 MH⁺
Masse moléculaire mesurée (Sphe-2S): 4502,71 MH⁺
Appariement des cystéines : Cysl-Cys5, Cys2-Cys4, Cys3-Cys6

Du fait de cette identité stricte, la sphéniscine-2 synthétique a été utilisée pour effectuer les études des propriétés biologiques de cette molécule et d'envisager l'étude de la structure tri-dimensionnelle de ce peptide.

### 4.3. Détermination du spectre d'activité de la sphéniscine-2.

Les activités antimicrobiennes exprimées en concentration minimale d'inhibition (CMI) de la sphéniscine-2 (concentrations comprises entre 0.2 µM et 100 µM) ont été déterminées contre des bactéries, des levures et des champignons en utilisant un test d'inhibition de croissance en milieu liquide dans des plaques de microtitration (pour les protocoles détaillés voir l'exemple 2.4. ainsi que Hétru et Bulet 1997).

L'effet bactéricide ou bactériostatique du peptide a été déterminé par étalement sur boîte de Pétri et dénombrement des colonies présentes après 24 h d'incubation.

Les souches de microorganismes utilisées sont les mêmes que certaines précédemment décrites dans la littérature (Lowenberger et al. 1995, 1999) auxquelles ont été ajoutées les souches suivantes (dons de chercheurs) : *Bacillus cereus* ATCC 11778 (Collection de l'Institut Pasteur, Paris), *Alcaligenes faecalis, Staphylococcus saprophyticus, S. haemolyticus* et *Nocardia astéroïdes* (Pr. Monteil et Piémont, Institut de Bactériologie, Université de Strasbourg, France), *Escherichia coli* SBS 363 (Dr. Boquet, Centre d'Etudes Nucléaires, Saclay, France), *Vibrio metshnikovii* et *V*. *anguillarum* (Dr. Bachère, IFREMER, Montpellier, France), *Candida albicans* IHEM 8060 (EntoMed, Strasbourg, France) et *C. tropicalis* (Dr. Koenig, Hôpital Civil, Strasbourg, France).

Le peptide contrôle MSI-94 est un don du Dr. M.A. Zasloff (Magainin Scientific Institute, Plymouth Meeting, Philadelphia) ; et le peptide thanatine est un don du Dr. Bulet (CNRS, UPR 9022, Strasbourg).

Le tableau 1 ci-après présente le spectre d'activité de la sphéniscine-2 de synthèse et de deux peptides contrôles. La concentration minimale d'inhibition de croissance (CMI) correspond à l'intervalle [a] - [b] où [a] est la plus forte concentration testée pour laquelle le microorganisme pousse, et [b] la plus faible concentration testée à partir de laquelle il y a 100% d'inhibition de croissance du microorganisme (Casteels et Tempst 1994). Les peptides contrôles sont MSI-94 (un peptide de la famille des mangainines, issue de la peau du batracien *Xenopus laevis,* et présentant un large spectre d'activité antimicrobienne (Maloy et Kari 1995) pour tester contre les bactéries et les levures ; et la thanatine (un peptide antifongique provenant de l'insecte *Podisus maculiventris ;* Fehlbaum et al. 1996) pour tester contre les champignons filamenteux. ND signifie que l'activité du peptide n'a pas été détectée aux concentrations testées, soit jusqu'à la concentration maximale de 100 µM.

**Tableau 1: Activité antimicrobienne de la sphéniscine-2 de synthèse et de peptides contrôles. Gammes de concentrations testées : sphéniscine-2 = 0,2M - 100µM ; MSI-94 (contrôle pour les bactéries et les levures) : 0,2µM - 10µM ; Thanatine (contrôle pour les champignons) : 0,4µM - 41,1µM).**

| Microorganismes | CMI (µM) | | Effet bactéricide de la sphéniscine-2 |
|---|---|---|---|
| | Sphéniscine | Antibiotique contrôle | |
| **Bactéries à Gram-positif** | | | |
| *Micrococcus luteus* | 1,5-3 | 0,63-1,25 | Oui |
| *Bacillus subtilis* | 0,8-1,5 | 1,25-2,5 | Oui |
| *B. cereus* ATCC 11778 | 3-6 | 1,25-2,5 | Oui |
| *B. megaterium* | 0,4-0,8 | 0,31-0,.63 | Oui |
| *Staphylococcus aureus* | 1,5-3 | 1,25-2,5 | Oui |
| *S. saprophyticus* | 1,5-3 | 0,63-1,25 | Non |
| *S. haemolyticus* | 1,5-3 | 0,63-1,25 | Oui |
| *Nocardia asteroïdes* | 0,8-1,5 | 1,25-2,5 | Oui |
| *Aerococcus viridans* | 0..4-0.8 | 1,25-2,5 | - |
| *Listeria monocytogenes* | 6-12 | 2,5-5 | - |

| **Bactéries à Gram-négatif** | | | |
|---|---|---|---|
| *Escherichia coli* SBS 363 | 0,8-1,5 | 1,25-2,5 | Non |
| *E. coli* 1106 | 1,.5-3 | 1.25-2..5 | Oui |
| *Enterobacter cloacae* | ND | 5-10 | - |
| *Salmonella typhimurium* | 6-12 | 2,5-5,0 | Non |
| *Klebsiella pneumoniae* | 50-100 | 2,5-5,0 | Non |
| *Pseudomonas aeruginosa* ATCC 82118 | 6-12 | 0,63-1,25 | Non |
| *Alcaligenes faecalis* | ND | 2,5-5 | - |
| *Vibrio metshnikovii* NCTC 8483 | 25-50 | - | Oui |
| *V. anguillarum* ATCC 19264 | 25-50 | - | Non |

| **Levures** | | | |
|---|---|---|---|
| *Candida glabrata* | >100 | ND | |
| *C. albicans* IHEM 8060 | 50-100 | 2,5-5,0 | |
| *C. tropicalis* | 1,.5-3 | 2,5 - 5,0 | |

| **Champignons** | | | |
|---|---|---|---|
| *Neurospora crassa* | 3-6 | 2..0-4.0 | |
| *Aspergillus, fumigatus* | 3-6 | close to 20 | |

| | | | |
|---|---|---|---|
| ND _{:} activité non détectée dans la gamme de concentrations testée. | | | |

### 4.4. Etude de la fonctionnalité de la sphéniscine-2 à différents pH.

Afin de déterminer si l'acidité modérée du contenu stomacal bien préservé (pH 4-6, Thouzeau et al. 2003) pouvait modifier l'efficacité de la sphéniscine in vivo, l'activité de la sphéniscine a été testée aux pH compris entre 4,2 et 6,1.

Des souches de microorganismes capables de pousser à de tels pH ont été sélectionnées au préalable : il s'agit de *Pseudomonas aeruginosa* et *d'Escherichia coli* 1106 (bactéries à Gram-négatif).

Les tests d'activité antimicrobienne ont été réalisés en milieu liquide dans des plaques de microtitration (pour les protocoles détaillés voir l'exemple 2.4. ainsi que Hétru and Bulet 1997).

Pour chaque test, le pH du milieu liquide utilisé a été ajusté avec de l'acide chlorhydrique.

Le tableau 2 ci-après présente l'effet du pH du milieu sur l'activité antimicrobienne de la sphéniscine-2 de synthèse. Deux souches de bactéries à Gram-négatif *(Pseudomonas aeruginosa* et *Escherichia coli* 1106) capables de pousser dans la gamme de pH à tester ont été sélectionnées. La gamme de pH testée couvre les variations de pH observées dans le contenu stomacal bien conservé du Manchot Royal incubant (Thouzeau et al. 2003).

**Table 2 : Effet du pH sur l'activité de la sphéniscine-2 de synthèse.**

| Microorganismes | pH | CMI (µM) après 48h d'incubation |
|---|---|---|
| *Escherichia coli* | 4,2 | 6 - 12 |
| *1106* | 5,2 | 25 - 50 |
| | 6,1 | 6 - 12 |
| | 7,2 | 1,5 - 3 |
| | | |
| *Pseudomonas* | 4,2 | > 100 |
| *aeruginosa* | 5,2 | ND |
| | 6,1 | ND |
| | 7,2 | 6 - 12 |

| | | |
|---|---|---|
| ND : activité non détectée dans la gamme de concentrations testée. | | |

### REFERENCES BIBLIOGRAPHIQUES

Bals, R., Goldman, M.J., & Wilson, J.M. (1998). Mouse beta-defensin 1 is a salt-sensitive antimicrobial peptide present in epithelia of the lung and urogenital tract. Infect Immun, 66, 1225-32.
Bals, R., Lang, C., Weiner, D.J., Vogelmeier, C., Welsch, U., & Wilson, J.M. (2001). Rhesus monkey (Macaca mulatta) mucosal antimicrobial peptides are close homologues of human molecules. Clin Diagn Lab Immunol, 8, 370-5.
Bals, R., Wang, X., Meegalla, R.L., Wattler, S., Weiner, D.J., Nehls, M.C., & Wilson, J.M. (1999). Mouse beta-defensin 3 is an inducible antimicrobial peptide expressed in the epithelia of multiple organs. Infect Immun, 67, 3542-7.
Bost, C.A., Georges, J.-Y., Guinet, C., Cherel, Y., Pütz, K., Charrassin, J.-B., Handrich, Y., Zorn, T., Lage, J., & Le Maho, Y. (1997). Foraging habitat and food intake of satellite-tracked king penguins during the autral summer at Crozet Archipelago. Marine Ecology Progress Series, 150, 21-33.
Bulet, P. (1999). Les peptides antimicrobiens de la drosophile. médecine/sciences, 15,23-29.
Casteels, P., & Tempst, P. (1994) Apidaecin-type peptide antibiotics function through a non-poreforming mechanism involving stereospecificity. Biochem Biophys Res Commun, 199, 339-345.
Conejo-Garcia J.R. et al. Site internet Antimicrobial séquences databank (AMSDb) http://www.bbcm.univ.trieste.it/-tossi/pagl.htm
Diamond, G., Jones, D.E., & Bevins, C.L. (1993). Airway epithelial cells are the site of expression of a mammalian antimicrobial peptide gene. Proc Natl Acad Sci U S A, 90, 4596-600.
Duits, L.A., Langermans, J.A., Paltansing, S., van der Straaten, T., Vervenne, R.A., Frost, P.A., Hiemstra, P.S., Thomas, A.W., & Nibbering, P.H. (2000). Expression of beta-defensin-1 in chimpanzee (Pan troglodytes) airways. J Med Primatol, 29, 318-23.
Duits, L.A, et al. Site internet Antimicrobial **séquences databank** (**AMSDb**) http://www.bbcm.univ.trieste.it/-tossi/pagl.htm
Fehlbaum, P., Bulet, P., Chernysh, S., Briand, J. P., Roussel, J. P., Letellier, L., Hetru, C., & Hoffmann, J. A. (1996) Structure-activity analysis of thanatin, a 21-residue inducible insect defense peptide with sequence homology to frog skin antimicrobial peptides. Proc Natl Acad Sci U S A, 96, 1221-1225.
Garcia, J.R., Jaumann, F., Schulz, S., Krause, A., Rodriguez-Jimenez, J., Forssmann, U., Adermann, K., Kluver, E., Vogelmeier, C., Becker, D., Hedrich, R., Forssmann, W.G., & Bals, R. (2001). Identification of a novel, multifunctional beta-defensin (human beta- defensin 3) with specific antimicrobial activity. Its interaction with plasma membranes of Xenopus oocytes and the induction of macrophage chemoattraction. Cell Tissue Res, 306, 257-64.
Garcia, J.R., Krause, A., Schulz, S., Rodriguez-Jimenez, F.J., Kluver, E., Adermann, K., Forssmann, U., Frimpong-Boateng, A., Bals, R., & Forssmann, W.G. (2001). Human beta-defensin 4: a novel inducible peptide with a specific salt- sensitive spectrum of antimicrobial activity. FASEB J, 15, 1819-21.
Gauthier-Clerc, M., Le Maho, Y., Clerquin, Y., Bost, C.-A., & Handrich, Y. (2002). Seabird reproduction in an unpredictable environment : how king penguins provide their young chicks with food. Marine Ecology Progress Series, in press*,*
Gauthier-Clerc, 1 M., Le Maho, Y., Clerquin, Y., Drault, S., & Handrich, Y. (2000). Penguin fathers preserve food for their chicks. Nature, 408, 928-9.
Harder, J., Bartels, J., Christophers, E., & Schroder, J.M. (2001). Isolation and characterization of human beta-defensin-3, a novel human inducible peptide antibiotic. J Biol Chem, 276, 5707-13.
Harder, J., Bartels, J., Christophers, E., & Schroder, J.M. (1997). A peptide antibiotic from human skin. Nature, 387, 861
Hétru, C., & Bulet, P. (1997). Strategies for the isolation and characterization of antimicrobial peptides of invertebrates. From Methods in Molecular Biology, vol. 78, W. M. Shafer (ed.) Humana Press Inc., Totowa, NJ
Hiratsuka, T., Nakazato, M., Date, Y., Ashitani, J., Minematsu, T., Chino, N., & Matsukura, S. (1998). Identification of human beta-defensin-2 in respiratory tract and plasma and its increase in bacterial pneumonia. Biochem Biophys Res Commun, 249, 943-7.
Huttner, K.M., Brezinski-Caliguri, D.J., Mahoney, M.M., & Diamond, G. (1998). Antimicrobial peptide expression is developmentally regulated in the ovine gastrointestinal tract. J Nutr, 128, 297S-299S.
Huttner, K.M., Kozak, C.A., & Bevins, C.L. (1997). The mouse genome encodes a single homolog of the antimicrobial peptide human beta-defensin 1. FEBS Lett, 413, 45-9.
Jia, H.P., Wowk, S.A., Schutte, B.C., Lee, S.K., Vivado, A., Tack, B.F., Bevins, C.L., & McCray, P.B. Jr. (2000). A novel murine beta -defensin expressed in tongue, esophagus, and trachea. J Biol Chem, 275, 33314-20.
Jones, D.E., & Bevins, C.L. (1992). Paneth cells of the human small intestine express an antimicrobial peptide gene. J Biol Chem, 267, 23216-25..
Kussmann, M., Nordhoff, E., Rahbek-Nielsen, H., Haebel, S., Rossel-Larsen, M., Jakobsen, L., Gobom, J., Mirgorodskaya, E., Kroll-Kristensen, A., Palm, L.,& Roepstorff P. (1997). Matrix-assisted Laser Desorption/Ionization Mass Spectrometry Sample Preparation Techniques. Designed for Various Peptide and Protein Analytes. J Mass Spectrom, 32, 593-601.
Lowenberger, C., Bulet, P., Charlet, M., Hetru, C., Hodgeman, B., Christensen, B. M., & Hoffmann, J. A. (1995) Insect immunity: isolation of three novel inducible antibacterial defensins from the vector mosquito, Aedes aegypti. Insect Biochem Mol Biol, 25, 867-73.
Lowenberger, C., Charlet, M., Vizioli, J., Kamal, S., Richman, A., Christensen, B. M., & Bulet, P. (1999) Antimicrobial activity spectrum, cDNA cloning, and mRNA expression of a newly isolated member of the cecropin family from the mosquito vector Aedes aegypti. J Biol Chem, 274, 20092-20097.
Mac Cormack, W.P., & Fraile, E.R. (1990). Bacterial flora of newly caught antarctic fish Notothenia neglecta. Polar Biology, 10, 413-417.
Maloy, W. L. , & Kari, U. P. (1995) Structure-activity studies on magainins and other host defense peptides; Biopolymers, 37, 105-122.
Mathews, M., Jia, H.P., Guthmiller, J.M., Losh, G., Graham, S., Johnson, G.K., Tack, B.F., & McCray, P.B.J. (1999). Production of beta-defensin antimicrobial peptides by the oral mucosa and salivary glands. Infect Immun, 67, 2740-5.
Minn, I., Kim, H.S., & Kim, S.C. (1998). Antimicrobial peptides derived from pepsinogens in the stomach of the bullfrog, Rana catesbeiana. Biochim Biophys Acta, 1407, 31-9.
Moore, K.S., Bevins, C.L., Brasseur, M.M., Tomassini, N., Turner, K., Eck, H., & Zasloff, M. (1991). Antimicrobial peptides in the stomach of Xenopus laevis. J Biol Chem, 266, 19851-7.
Morrison, G.M., Davidson, D.J., & Dorin, J.R. (1999). A novel mouse beta defensin, Defβ2, which is upregulated in the airways by lipopolysaccharide. FEBS Lett, 442, 112-6.
O'Neil, D.A., Cole, S.P., Martin-Porter, E., Housley, M.P., Liu, L., Ganz, T., & Kagnoff, M.F. (2000). Regulation of human beta-defensins by gastric epithelial cells in response to infection with Helicobacter pylori or stimulation with interleukin-1. Infect Immun, 68, 5412-5.
Perregaux, D.G., Bhavsar, K., Contillo, L., Shi, J., & Gabel, C.A. (2002). Antimicrobial Peptides Initiate IL-lbeta Posttranslational Processing: A Novel Role Beyond Innate Immunity. J Immunol, 168, 3024-32.
Schonwetter, B.S., & et al. (1995). Epithelia antibiotics induced at sites of inflammation. Sciences, 265, 1645-1648.
Schröder, J.M. (1999). Epithelial antimicrobial peptides: innate local host response elements. Cell Mol Life Sci, 56, 32-46.
Soucek, Z., & Mushin, R. (1970). Gastrointestinal bacteria of certain antarctic birds and mammals. Applied Microbiology, 20, 561-566.
Tarver, A.P., Clark, D.P., Diamond, G., Russell, J.P., Erdjument-Bromage, H., Tempst, P., Cohen, K.S., Jones, D.E., Sweeney, R.W., Wines, M., Hwang, S., & Bevins, C.L. (1998). Enteric beta-defensin: molecular cloning and characterization of a gene with inducible intestinal epithelial cell expression associated with Cryptosporidium parvum infection. Infect Immun, 66, 1045-56.
Thouzeau, C., Froget, G., Monteil, H., Le Maho, Y. & Harf-Monteil, C. (2003) Evidence of stress in bacteria associated with long-term preservation of food in the stomach of incubating king penguins (Aptenodytes patagonicus). Polar Biol, 26, 115-123.
Wang, Y., Knoop, F.C., Remy-Jouet, I., Delarue, C., Vaudry, H., & Conlon, J.M. (1998). Antimicrobial peptides of the brevinin-2 family isolated from gastric tissue of the frog, Rana esculenta. Biochem Biophys Res Commun, 253, 600-3.
Yamaguchi, Y., Fukuhara, S., Nagase, T., Tomita, T., Hitomi, S., Kimura, S., Kurihara, H., & Ouchi, Y. (2001). A novel mouse beta-defensin, mBD-6, predominantly expressed in skeletal muscle. J Biol Chem, 276, 31510-4.
Zhang, G., Wu, H., Shi, J., Ganz, T., Ross, C.R., & Blecha, F. (1998). Molecular cloning and tissue expression of porcine beta-defensin-1. FEBS Lett, 424, 37-40.
Zhao, C., Nguyen, T., Liu, L., Shamova, O., Brogden, K., & Lehrer, R.I. (1999). Differential expression of caprine beta-defensins in digestive and respiratory tissues. Infect Immun, 67, 6221-4.
Zhao, C., Nguyen, T., Liu, L., Sacco, R.E., Brogden, K.A., & Lehrer, R.I. (2001). Gallinacin-3, an inducible epithelial beta-defensin in the chicken. Infect Immun, 69, 2684-91.

### LISTAGE DE SEQUENCES

<110> Centre National de la Recherche Scientifique
<120> PEPTIDES AYANT DES PROPRIETES ANTIMICROBIENNES ET LES COMPOSITIONS LES CONTENANT NOTAMMENT POUR LA CONSERVATION DES ALIMENTS
<130> 27059FR CNRS 28 juin 2002
<140> PCT/FR03/XXXXX
   <141> 01-07-03
<150> FR02/08207
   <151> 01-07-02
<160> 16
   <170> WORD® 2000
<210> 1
   <211> 15
   <212> PRT
   <213> Sphéniscidés
   <220>
   <221>
   <222> 1
   <223>
   Xaa représente un groupe -NH₂ ou un reste peptidique de 1 à 16 acides aminés et de préférence de 4 acides aminés,
   Xaa répond à la formule suivante : -Xaal-Xaa2- (SEQ ID NO.2)
<220>
   <221>
   <222> 3
   <223>
   Xab représente un reste peptidique de 1 à 6 acides aminés, de préférence 6 acides aminés
   Xab répond à la formule suivante : -Xab1-Xab2-Xab3-Xab4-Xab5- (SEQ ID NO.3)
<220>
   <221>
   <222> 5
   <223>
   Xac représente un reste peptidique comprenant de 1 à 4 acides aminés, de préférence 4 acides aminés
   Xac répond à la formule suivante : Xac1-Xac2-Xac3-Xac4 (SEQ ID NO.5)
<220>
   <221>
   <222> 7
   <223>
   Xad représente un reste peptidique comprenant de 1 à 9 acides aminés, de préférence 9 acides aminés, avantageusement Xad répond à la séquence suivante: -Xad1-Xad2-Xad3-Xad4-Xad5-Xad6-Xad7-Xad8-Xad9- (SEQ ID NO.6),
<220>
   <221>
   <222> 9
   <223>
   Xae représente un reste peptidique comprenant de 1 à 6 acides aminés, de préférence 5 acides aminés, avantageusement Xae répond à la séquence suivante : -Xae1-Xae2-Xae3-Xae4-Xae5- (SEQ ID NO.7)
<220>
   <221>
   <222> 12
   <223>
   Xaf représente un groupe -OH ou -CONH₂ ou encore un reste peptidique de 1 à 14 acides aminés, de préférence 4 acides aminés, avantageusement Xaf répond à la séquence suivante : -Xaf1-Xaf2-Xaf3-Xaf4- (SEQ ID NO.8)
<220>
   <221>
   <222> 2
   <223>
   Ca1 représente un acide aminé lié à l'un quelconque des autres Ca2, Ca3, Ca4, Ca5 et Ca6, avantageusement Ca1 est un acide aminé soufré de type cystéine et préférentiellement une cystéine.
<220>
   <221>
   <222> 4
   <223>
   Ca2 représente un acide aminé lié à l'un quelconque des autres Ca1, Ca3, Ca4, Ca5 et Ca6, avantageusement Ca2 est un acide aminé soufré de type cystéine et préférentiellement une cystéine.
<220>
   <221>
   <222> 6
   <223>
   Ca3 représente un acide aminé lié à l'un quelconque des autres Ca1, Ca2, Ca4, Ca5 et Ca6, avantageusement Ca3 est un acide aminé soufré de type cystéine et préférentiellement une cystéine.
<220>
   <221>
   <222> 8
   <223>
   Ca4 représente un acide aminé lié à l'un quelconque des autres Ca1, Ca2, Ca3, Ca5 et Ca6, avantageusement Ca4 est un acide aminé soufré de type cystéine et préférentiellement une cystéine.
<220>
   <221>
   <222> 10
   <223>
   Ca5 représente un acide aminé lié à l'un quelconque des autres Ca1, Ca2, Ca3, Ca4 et Ca6, avantageusement Ca5 est un acide aminé soufré de type cystéine et préférentiellement une cystéine.
<220>
   <221>
   <222> 11
   <223>
   Ca6 représente un acide aminé lié à l'un quelconque des autres Ca1, Ca2, Ca3, Ca4 et Ca5, avantageusement Ca6 est un acide aminé soufré de type cystéine et préférentiellement une cystéine.
<400> 1
   Xaa Ca1 Xab Ca2 Xac Ca3 Xad Ca4 Xae Ca5 Ca6 Xaf
<210> 2
   <211> 2
   <212> PRT
   <213> Sphéniscidés
   <220>
   <221>
   <222> 1
   <223>
   Xaa1 représente un groupe -NH₂ ou un reste peptidique de 1 à 13 acides aminés
<220>
   <221>
   <222> 2
   <223>
   Xaa2 représente un reste peptidique de 3 acides aminés choisis parmi les acides aminés hydrophobes ou apolaires ;
<400> 2
   Xaa1 Xaa2
<210> 3
   <211> 5
   <212> PRT
   <213> Sphéniscidés
<220>
   <221>
   <222> 1
   <223>
   Xab1 est choisi dans le groupe comprenant les acides aminés basiques, les acides aminés polaires chargés négativement, les acides aminés polaires petits non chargés, les acides aminés polaires larges non chargés et les acides aminés hydrophobes ou apolaires
<220>
   <221>
   <222> 2
   <223>
   Xab2 est choisi dans le groupe comprenant les acides aminés basiques, les acides aminés polaires chargés négativement, les acides aminés polaires petits non chargés, les acides aminés polaires larges non chargés et les acides aminés hydrophobes ou apolaires
<220>
   <221>
   <222> 3
   <223>
   Xab3 représente un reste peptidique répondant à la formule suivante -Xab3.1-Xab3.2- (SEQ ID NO.4)
<220>
   <221>
   <222> 4
   <223>
   Xab4 est choisi dans le groupe comprenant les acides aminés basiques, les acides aminés polaires chargés négativement, les acides aminés polaires petits non chargés, les acides aminés polaires larges non chargés et les acides aminés hydrophobes ou apolaires
<220>
   <221>
   <222> 5
   <223>
   Xab5 est choisi dans le groupe comprenant les acides aminés basiques, les acides aminés polaires chargés négativement, les acides aminés polaires petits non chargés, les acides aminés polaires larges non chargés et les acides aminés hydrophobes ou apolaires
<400> 3
   Xab1 Xab2 Xab3 Xab4 Xab5
<210> 4
   <211> 2
   <212> PRT
   <213> Sphéniscidés
   <220>
   <221>
   <222> 1
   <223>
   Xab3.1 est choisi dans le groupe comprenant les acides aminés basiques, les acides aminés polaires chargés négativement, les acides aminés polaires petits non chargés, les acides aminés polaires larges non chargés et les acides aminés hydrophobes ou apolaires
<220>
   <221>
   <222> 2
   <223>
   Xab3.2 est choisi dans le groupe comprenant les acides aminés basiques, les acides aminés polaires chargés négativement, les acides aminés polaires petits non chargés, les acides aminés polaires larges non chargés et les acides aminés hydrophobes ou apolaires
<400> 4
   Xab3.1 Xab3.2
<210> 5
   <211> 4
   <212> PRT
   <213> Sphéniscidés
   <220>
   <221>
   <222> 1
   <223> Xac1 est choisi dans le groupe comprenant les acides aminés basiques, les acides aminés polaires petits non chargés, les acides aminés hydrophobes ou apolaires et les acides aminés polaires larges non chargés
<220>
   <221>
   <222> 2
   <223> Xac2 est choisi dans le groupe comprenant les acides aminés basiques, les acides aminés polaires petits non chargés, les acides aminés hydrophobes ou apolaires et les acides aminés polaires larges non chargés
<220>
   <221>
   <222> 3
   <223> Xac3 est choisi dans le groupe comprenant les acides aminés basiques, les acides aminés polaires petits non chargés, les acides aminés hydrophobes ou apolaires et les acides aminés polaires larges non chargés
<220>
   <221>
   <222> 4
   <223> Xac4 est choisi dans le groupe comprenant les acides aminés basiques, les acides aminés polaires petits non chargés, les acides aminés hydrophobes ou apolaires et les acides aminés polaires larges non chargés
<400> 5
   Xac1 Xac2 Xac3 Xac4
<210> 6
   <211> 9
   <212> PRT
   <213> Sphéniscidés
   <220>
   <221>
   <222> 1
   <223>
   Xad1 est choisi dans le groupe comprenant les acides aminés basiques, les acides aminés polaires petits non chargés, les acides aminés hydrophobes ou apolaires, les acides aminés polaires larges non chargés et les acides aminés polaires chargés négativement
<220>
   <221>
   <222> 2
   <223>
   Xad2 est choisi dans le groupe comprenant les acides aminés basiques, les acides aminés polaires petits non chargés, les acides aminés hydrophobes ou apolaires, les acides aminés polaires larges non chargés et les acides aminés polaires chargés négativement.
<220>
   <221>
   <222> 3
   <223>
   Xad3 est choisi dans le groupe comprenant les acides aminés basiques, les acides aminés polaires petits non chargés, les acides aminés hydrophobes ou apolaires, les acides aminés polaires larges non chargés et les acides aminés polaires chargés négativement
<220>
   <221>
   <222> 4
   <223>
   Xad4 est choisi dans le groupe comprenant les acides aminés basiques, les acides aminés polaires petits non chargés, les acides aminés hydrophobes ou apolaires, les acides aminés polaires larges non chargés et les acides aminés polaires chargés négativement
<220>
   <221>
   <222> 5
   <223>
   Xad5 est choisi dans le groupe comprenant les acides aminés basiques, les acides aminés polaires petits non chargés, les acides aminés hydrophobes ou apolaires, les acides aminés polaires larges non chargés et les acides aminés polaires chargés négativement
<220>
   <221>
   <222> 6
   <223>
   Xad6 est choisi dans le groupe comprenant les acides aminés basiques, les acides aminés polaires petits non chargés, les acides aminés hydrophobes ou apolaires, les acides aminés polaires larges non chargés et les acides aminés polaires chargés négativement
<220>
   <221>
   <222> 7
   <223>
   Xad7 est choisi dans le groupe comprenant les acides aminés basiques, les acides aminés polaires petits non chargés, les acides aminés hydrophobes ou apolaires, les acides aminés polaires larges non chargés et les acides aminés polaires chargés négativement
<220>
   <221>
   <222> 8
   <223>
   Xad8 est choisi dans le groupe comprenant les acides aminés basiques, les acides aminés polaires petits non chargés, les acides aminés hydrophobes ou apolaires, les acides aminés polaires larges non chargés et les acides aminés polaires chargés négativement
<220>
   <221>
   <222> 9
   <223>
   Xad9 est choisi dans le groupe comprenant les acides aminés basiques, les acides aminés polaires petits non chargés, les acides aminés hydrophobes ou apolaires, les acides aminés polaires larges non chargés et les acides aminés polaires chargés négativement
<400> 6
   Xad1 Xad2 Xad3 Xad4 Xad5 Xad6 Xad7 Xad8 Xad9
<210> 7
   <211> 5
   <212> PRT
   <213> Sphéniscidés
   <220>
   <221>
   <222> 1
   <223>
   Xae1 est choisi dans le groupe comprenant les acides aminés basiques, les acides aminés polaires petits non chargés, les acides aminés hydrophobes ou apolaires, les acides aminés polaires larges non chargés et les acides aminés polaires chargés négativement.
<220>
   <221>
   <222> 2
   <223>
   Xae2 est choisi dans le groupe comprenant les acides aminés basiques, les acides aminés polaires petits non chargés, les acides aminés hydrophobes ou apolaires, les acides aminés polaires larges non chargés et les acides aminés polaires chargés négativement.
<220>
   <221>
   <222> 3
   <223>
   Xae3 est choisi dans le groupe comprenant les acides aminés basiques, les acides aminés polaires petits non chargés, les acides aminés hydrophobes ou apolaires, les acides aminés polaires larges non chargés et les acides aminés polaires chargés négativement.
<220>
   <221>
   <222> 4
   <223>
   Xae4 est choisi dans le groupe comprenant les acides aminés basiques, les acides aminés polaires petits non chargés, les acides aminés hydrophobes ou apolaires, les acides aminés polaires larges non chargés et les acides aminés polaires chargés négativement.
<220>
   <221>
   <222> 5
   <223>
   Xae5 est choisi dans le groupe comprenant les acides aminés basiques, les acides aminés polaires petits non chargés, les acides aminés hydrophobes ou apolaires, les acides aminés polaires larges non chargés et les acides aminés polaires chargés négativement.
<400> 7
   Xae1 Xae2 Xae3 Xae4 Xae5
<210> 8
   <211> 4
   <212> PRT
   <213> Sphéniscidés
   <220>
   <221>
   <222> 1
   <223>
   Xaf1 est choisi dans le groupe comprenant les acides aminés basiques, les acides aminés polaires petits non chargés, les acides aminés hydrophobes ou apolaires, les acides aminés polaires larges non chargés et les acides aminés polaire chargé négativement
<220>
   <221>
   <222> 2
   <223>
   Xaf2 est choisi dans le groupe comprenant les acides aminés basiques, les acides aminés polaires petits non chargés, les acides aminés hydrophobes ou apolaires, les acides aminés polaires larges non chargés et les acides aminés polaire chargé négativement
<220>
   <221>
   <222> 3
   <223>
   Xaf3 est choisi dans le groupe comprenant les acides aminés basiques, les acides aminés polaires petits non chargés, les acides aminés hydrophobes ou apolaires, les acides aminés polaires larges non chargés et les acides aminés polaire chargé négativement
<220>
   <221>
   <222> 4
   <223>
   Xaf4 est choisi dans le groupe comprenant les acides aminés basiques, les acides aminés polaires petits non chargés, les acides aminés hydrophobes ou apolaires, les acides aminés polaires larges non chargés et les acides aminés polaire chargé négativement
<400> 8
   Xaf1 Xaf2 Xaf3 Xaf4
<210> 9
   <211> 3
   <212> PRT
   <213> Sphéniscidés
   <220>
   <221>
   <222> 1
   <223>
   Xac1 est choisi dans le groupe comprenant les acides aminés basiques, les acides aminés hydrophobes ou apolaires, les acides aminés polaires larges non chargés, de préférence Xac1 est un acide aminé hydrophobe ou apolaire et tout préférentiellement l'alanine (Ala).
<220>
   <221>
   <222> 3
   <223>
   Xac3 est choisi dans le groupe comprenant les acides aminés basiques, les acides aminés hydrophobes ou apolaires, les acides aminés polaires petits non chargés, les acides aminés polaires larges non chargés, de préférence Xac3 est un acide aminé hydrophobe ou apolaire et tout préférentiellement la glycine (Gly).
<400> 9
   Xac1 His Xac3
<210> 10
   <211> 3
   <212> PRT
   <213> Sphéniscidés
   <220>
   <221>
   <222> 1
   <223>
   Xac1 est choisi dans le groupe comprenant les acides aminés basiques, les acides aminés hydrophobes ou apolaires, les acides aminés polaires larges non chargés, de préférence Xac1 est un acide aminé hydrophobe ou apolaire et tout préférentiellement l'alanine (Ala).
<220>
   <221>
   <222> 3
   <223>
   Xac3 est choisi dans le groupe comprenant les acides aminés basiques, les acides aminés hydrophobes ou apolaires, les acides aminés polaires petits non chargés, les acides aminés polaires larges non chargés, de préférence Xac3 est un acide aminé hydrophobe ou apolaire et tout préférentiellement la glycine (Gly).
<400> 10
   Xac1 Arg Xac3
<210> 11
   <211> 3
   <212> PRT
   <213> Sphéniscidés
   <220>
   <221>
   <222> 1
   <223>
   Xad1 est choisi dans le groupe comprenant les acides aminés basiques, les acides aminés hydrophobes ou apolaires, les acides aminés polaires larges non chargés, de préférence Xad1 est un acide aminé basique et tout préférentiellement l'arginine (Arg).
<220>
   <221>
   <222> 3
   <223>
   Xad3 est choisi dans le groupe comprenant les acides aminés hydrophobes ou apolaires, les acides aminés polaires petits non chargés, les acides aminés basiques, les acides aminés polaires larges non chargés, de préférence un acide aminé hydrophobe ou apolaire, de préférence Xad3 est la proline ou l'hydroxyproline et tout préférentiellement la proline (Pro).
<400> 11
   Xad1 Pro Xad3
<210> 12
   <211> 3
   <212> PRT
   <213> Sphéniscidés
   <220>
   <221>
   <222> 1
   <223>
   Xae1 est choisi dans le groupe comprenant les acides aminés basiques, les
   acides aminés hydrophobes ou apolaires, les acides aminés polaires larges non chargés, les acides aminés polaires petits non chargés, de préférence Xae1 est un acide aminé polaire petit non chargé et tout préférentiellement 1a serine (Ser).
<220>
   <221>
   <222> 3
   <223>
   Xae3 est choisi dans le groupe comprenant les acides aminés basiques, les acides aminés hydrophobes ou apolaires, les acides aminés polaires larges non chargés, les acides aminés polaires petits non chargés, les acides aminés polaires chargés négativement, de préférence un acide aminé hydrophobe ou apolaire et tout préférentiellement la phénylalanine (Phe).
<400> 12
   Xae1 Gln Xae3
<210> 13
   <211> 3
   <212> PRT
   <213> Sphéniscidés
   <220>
   <221>
   <222> 1
   <223>
   Xaf1 est choisi dans le groupe comprenant les acides aminés basiques, les acides aminés hydrophobes ou apolaires, de préférence Xaf1 est un acide aminé basique et tout préférentiellement l'arginine (Arg).
<220>
   <221>
   <222> 3
   <223>
   Xaf3 est choisi dans le groupe comprenant les acides aminés basiques, les acides aminés hydrophobes ou apolaires, les acides aminés polaires larges non chargés, de préférence Xaf3 est un acide aminé hydrophobe ou apolaire et tout préférentiellement la valine (Val).
<400> 13
   Xaf1 Val Xaf3
<210> 14
   <211> 38
   <212> PRT
   <213> Sphéniscidés
   <220>
   <221> DISULFID
   <222> (5)..(33)
   <223> Pont disulfure entre les cystéines en positions 5 et 33.
<220>
   <221> DISULFID
   <222> (12)..(27)
   <223> Pont disulfure entre les cystéines en positions 12 et 27.
<220>
   <221> DISULFID
   <222> (17)..(34)
   <223> Pont disulfure entre les cystéines en positions 17 et 34.
<400> 14
   Ser Phe Gly Leu Cys Arg Leu Arg Arg Gly Phe Cys Ala His Gly Arg
   1               5                   10                  15
   Cys Arg Phe Pro Ser Ile Pro Ile Gly Arg Cys Ser Arg Phe Val Gln
               20                  25                  30
   Cys Cys Arg Arg Val Trp
           35
<210> 15
   <211> 38
   <212> PRT
   <213> Sphéniscidés
   <220>
   <221> DISULFID
   <222> (5)..(33)
   <223> Pont disulfure entre les résidus en positions 5 et 33
<220>
   <221> DISULFID
   <222> (12)..(27)
   <223> Pont disulfure entre les résidus en positions 12 et 27.
<220>
   <221> DISULFID
   <222> (17)..(34)
   <223> Pont disulfure entre les résidus en positions 17 et 34.
<400> 15
   Ser Phe Gly Leu Cys Arg Leu Arg Arg Gly Phe Cys Ala Arg Gly Arg
   1               5                   10                  15
   Cys Arg Phe Pro Ser Ile Pro Ile Gly Arg Cys Ser Arg Phe Val Gln
               20                  25                  30
   Cys Cys Arg Arg Val Trp
           35

## Revendications

1. Peptide ou un analogue de sphéniscine qui présente une activité antimicrobienne et comprend trois liaisons intra-moléculaires, et répond à la formule (II) suivante : 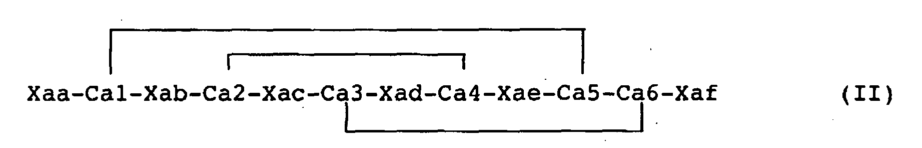 Dans laquelle :
- Xaa répond à la formule suivante : -Xaal-Xaa2- (SEQ ID NO.2), où Xaa1 représente un groupe -NH₂ ou un reste peptidique de 1 à 13 acides aminés, et Xaa2 est Ser-Phe-Gly-Leu;
- Xab répond à la formule suivante : -Xabl-Xab2-Xab3-Xab4-Xab5- (SEQ ID NO.3), et Xab3 représente un reste peptidique répondant à la formule suivante : -Xab3.1-Xab3.2- (SEQ ID NO.4),
- Xac répond à la formule suivante : -Xacl-Xac2-Xac3-Xac4- (SEQ ID NO.5), dans laquelle Xac2 est His ou Arg
- Xad à la séquence suivante : -Xad1-Xad2-Xad3-Xad4-Xad5-Xad6-Xad7-Xad8-Xad9- (SEQ ID NO.6), laquelle Xad2 est Pro ou Phe et Xad3 est Pro
- Xae répond à la séquence suivante : -Xael-Xae2-Xae3-Xae4-Xae5- (SEQ ID NO.7), dans laquelle Xae2 est Gln ou Arg et Xae5 est Gln ;
- Xaf répond à la séquence suivante : -Xaf1-Xaf2-Xaf3-Xaf4- (SEQ ID NO.8) et Xaf2 et/ou Xaf3 est Val; et - Ca1, Ca2, Ca3, Ca4, Ca5 et Ca6, sont des acides aminés soufrés.

2. Peptide selon la revendication 1, dans lequel :
Xaa est Ser-Phe-Gly-Leu,
Xab est Arg-Leu-Arg-Arg-Gly-Phe,
Xac est Ala-Xac2 -Gly-Arg,
Xad est Arg-Phe-Pro-Ser-Ile-Pro-Ile-Gly-Arg,
Xae est Ser-Arg-Phe-Val-Gln,
Xaf est Arg-Arg-Val-Trp, et
Xac2 est His ou Arg.

3. Composition pharmaceutique, agroalimentaire ou agronomique comprenant comme agent actif au moins un peptide selon l'une quelconque des revendications 1 à 2.

4. Composition selon la revendication 3, **caractérisée en ce qu'**elle est utile pour la conservation des aliments.

5. Composition selon la revendication 4, **caractérisée en ce qu'**elle est utile pour prévenir et/ou traiter une infection bactérienne et/ou fongique, chez l'homme, l'animal ou les plantes.

6. Polynucléotide **caractérisé en ce qu'**il code pour un peptide selon l'une quelconque des revendications 1 à 2.

7. Molécule d'acide nucléique, comme un vecteur, comprenant au moins un polynucléotide selon la revendication 6.

8. Hôte, comme une cellule animale ou végétale ou encore un procaryote, comprenant une molécule d'acide nucléique selon la revendication 7.

## Claims

1. Peptide or a spheniscin analogue which has an antibiotic activity and presents three intramolecular bonds responding to the sequence of formula (II) below 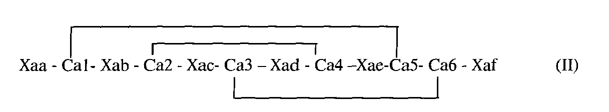 In which:
- Xaa responds to the following formula: -Xaal-Xaa2- (SEQ ID No. 2) wherein which Xaa1 represents an -NH₂ group or a peptide residue of 1 to 13 amino acids and Xaa2 is Ser-Phe-Gly-Leu;
- Xab responds to the following formula: -Xab1-Xab2-Xab3-Xab4-Xab5-(SEQ ID No. 3), and Xab3 represents a peptide residue responding to the following formula: -Xab3.1-Xab3.2- (SEQ ID No. 4)
- Xac responds to the following formula: -Xac1-Xac2-Xac3-Xac4- (SEQ ID No.5) wherein Xac2 is His or Arg
- Xad responds to the following sequence: -Xad1-Xad2-Xad3-Xad4-Xad5-Xad6-Xad7-Xad8-Xad9- (SEQ ID No. 6) wherein Xad2 is Pro or Phe et Xad3 is Pro;
- Xae responds to the following sequence: -Xae1-Xae2-Xae3-Xae4-Xae5-(SEQ ID No. 7) wherein Xae2 is Gln or Arg and Xae5 is Gln;
- Xaf responds to the following sequence: -Xaf1-Xaf2-Xaf3-Xaf4- (SEQ ID No. 8) and Xaf2 and/or Xaf3 is Val; and
- Ca1, Ca2, Ca3, Ca4, Ca5 and Ca6 sulfur-containing amino acids.

2. Peptide according to claim 1, wherein
- Xaa is Ser-Phe-Gly-Leu,
- Xab is Arg-Leu-Arg-Arg-Gly-Phe,
- Xac is Ala-Xac2-Gly-Arg,
- Xad is Arg-Phe-Pro-Ser-Ile-Pro-Ile-GlyArg,
- Xae is Ser-Arg-Phe-Val-Gln,
- Xaf is Arg-Arg-Val-Trp, and
- Xac2 is His or Arg.

3. Pharmaceutical, food processing or agricultural composition comprising as active agent at least one peptide according to any one of claims 1 to 2.

4. Composition according to claim 3, **characterized in that** it is useful for the preservation of foodstuffs.

5. Composition according to claim 4, **characterized in that** it is useful for preventing and/or treating a bacterial and/or fungal infection in humans, animals or plants.

6. Polynucleotide, **characterized in that** it codes for a peptide according to any one of claims 1 to 2.

7. Nucleic acid molecule as a vector comprising at least one polynucleotide according to claim 6.

8. Host, such as an animal or plant cell or a prokaryote, comprising a nucleic acid molecule according to claim 7.

## Patentansprüche

1. Peptid oder ein Analogon von Spheniscin, das eine antimikrobielle Aktivität aufweist und drei intramolekuläre Verbindungen umfasst sowie der folgenden Formel (II) entspricht: 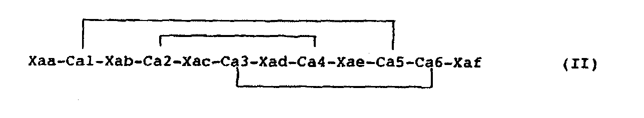 in der:
- Xaa der folgenden Formel entspricht: -Xaa1-Xaa2-(SEQ ID Nr. 2), wobei Xaa1 eine -NH₂-Gruppe oder einen Peptidrest mit 1 bis 13 Aminosäuren darstellt und Xaa2 = Ser-Phe-Gly-Leu ist;
- Xab der folgenden Formel entspricht: -Xab1-Xab2-Xab3-Xab4-Xab5-(SEQ ID Nr.3), und Xab3 einen Peptidrest darstellt, der der folgenden Formel entspricht: Xab3.1-Xab3.2- (SEQ ID NO.4),
- Xac der folgenden Formel entspricht: -Xac1-Xac2-Xac3-Xac4- (SEQ ID Nr. 5), wobei Xac2 = His oder Arg ist
- Xad der folgenden Sequenz entspricht: -Xad1-Xad2-Xad3-Xad4-Xad5-Xad6-Xad7-Xad8-Xad9- (SEQ ID Nr. 6), wobei Xad2 = Pro oder Phe und Xad3 = Pro ist
- Xae der folgenden Sequenz entspricht: -Xae1-Xae2-Xae3-Xae4-Xae5- (SEQ ID Nr. 7), wobei Xae2 = Gln oder Arg und Xae5 = Gln ist;
- Xaf der folgenden Sequenz entspricht: -Xaf1-Xaf2-Xaf3-Xaf4- (SEQ ID Nr.8) und Xaf2 und / oder Xaf3 = Val ist; und
- Ca1, Ca2, Ca3, Ca4, Ca5 und Ca6 geschwefelte Aminosäuren sind.

2. Peptid nach Anspruch 1, wobei:
Xaa = Ser-Phe-Gly-Leu ist,
Xab = Arg-Leu-Arg-Arg-Gly-Phe ist,
Xac = Ala-Xac2-Gly-Arg ist,
Xad = Arg-Phe-Pro-Ser-Ile-Pro-Ile-Gly-Arg ist,
Xae = Ser-Arg-Phe-Val-Gln ist,
Xaf = Arg-Arg-Val-Trp ist und
Xac2 = His oder Arg ist.

3. Pharmazeutische, Agrar-Nahrungsmittel- oder landwirtschaftliche Zusammensetzung, umfassend als Wirkstoff mindestens ein Peptid nach einem der Ansprüche 1 bis 2.

4. Zusammensetzung nach Anspruch 3, **dadurch gekennzeichnet, dass** sie für die Konservierung von Nahrungsmitteln nützlich ist.

5. Zusammensetzung nach Anspruch 4, **dadurch gekennzeichnet, dass** sie nützlich ist, um eine bakterielle und / oder Pilz-Infektion beim Menschen, bei Tieren oder Pflanzen vorzubeugen und / oder zu behandeln.

6. Polynukleotid, **dadurch gekennzeichnet, dass** es für ein Peptid nach einem der Ansprüche 1 bis 2 codiert.

7. Nukleinsäuremolekül als ein Vektor, umfassend mindestens ein Polynukleotid nach Anspruch 6.

8. Wirt als eine tierische oder pflanzliche Zelle oder auch ein Prokaryot, umfassend ein Nukleinsäuremolekül nach Anspruch 7.
